# EUROPEAN PATENT APPLICATION

(11) **EP 4 835 053 A1**
(43) Date of publication of application: **04.11.2026**
(21) Application number: 24911231.9
(22) Date of filing: 25.12.2024
(51) Int. Cl.: C12N 5/04, A61K 36/8984, A61K 8/99, A61Q 19/08

(54) **DENDROBIUM EXOSOME, PREPARATION METHOD THEREFOR, AND APPLICATION THEREOF**

(30) Priority: 29.12.2023 CN 202311867701; 24.05.2024 CN 202410661370
(71) Applicant: Cellular Biopharma (Shanghai), Ltd., Shanghai 201210 (CN)
(72) Inventor: LI, Ping, Shanghai 201210 (CN); ZHANG, Ye, Shanghai 201210 (CN); ZHAO, Bo, Shanghai 201210 (CN); ZHANG, Zeyi, Shanghai 201210 (CN); SHEN, Meiping, Shanghai 201210 (CN); LU, Tianlun, Shanghai 201210 (CN)
(74) Representative: EIP
(86) International application number: PCT/CN2024/142293
(87) International publication number: WO 2025/140308

(57) **Abstract**

A method for preparing a dendrobium exosome, the dendrobium exosome prepared by the method, and application of the dendrobium exosome. Specifically, the preparation method for the dendrobium plant exosome comprises the step of layer-by-layer filtration, wherein the step of layer-by-layer filtration sequentially comprises deep filtration and any membrane filtration.

## Description

### TECHNICAL FIELD

The present disclosure relates to a method for extracting exosomes from plants, such as from Dendrobium, and plant exosomes extracted by the method and application thereof. Specifically, the preparation method for the plant exosome comprises the step of layer-by-layer filtration, wherein the step of layer-by-layer filtration sequentially comprises deep filtration and membrane filtration.

### BACKGROUND ART

Exosomes are nanoparticles having a phospholipid bilayer secreted by cells, with a diameter usually of 30-200 nm, and contain abundant proteins, nucleic acid substances, and the like. Exosomes can transfer active substances such as proteins, mRNA, and microRNA between cells, participate in many important physiological and pathological processes, and their unique effects have received increasing attention in the industry.

At present, exosomes are mainly obtained from materials such as milk-derived samples, various cell culture supernatants, and exudates of tumor tissues. However, materials for obtaining exosomes from sources such as cell culture supernatants are limited, the cost is relatively high, the obtained exosome content is low, and the cost after purification is even higher, which restricts large-scale industrial production of exosomes.

Various plants are also important sources of exosomes, and the extraction rate of plant-derived exosome-like nanoparticles (Plant derived exosome-like nanoparticles, PEN) is far higher than that of mammalian cell cultures, indicating their economic effectiveness as nanofactories ^{[1]}. Most importantly, recent studies have shown that plant exosomes can be absorbed by intestinal macrophages and exert effects of intercellular communication and immune regulation ^{[2-3]}.

Correct extraction and purification methods are crucial for the analysis of exosomes, because they usually exist in matrices that are too complex. Research on exosome extraction methods needs not only to investigate the specificity and purity of extracts, but also to consider the simplicity of operation and scale efficiency. At present, methods more commonly used for isolating exosomes comprise ultracentrifugation method, polymer precipitation method, gel-exclusion method, antibody affinity precipitation method, density gradient centrifugation method, membrane filtration, and the like. However, each method has its defects; for example, ultracentrifugation method is complicated in operation, technically difficult, and time-consuming, the isolated exosomes may be damaged due to high-speed centrifugation and contain a large amount of miscellaneous proteins; exosomes extracted by polymer precipitation method lack specificity and selectivity and have low purity; gel-exclusion method has relatively high technical difficulty, but it is difficult to ensure that the isolated exosomes are sterile, is time-consuming, and is difficult to scale up; immunoaffinity chromatography usually increases the purity of samples but reduces efficiency and recovery rate; while membrane filtration improves efficiency and recovery rate but reduces specificity and purity of samples^{[4]}.

Usually, mammalian-derived exosomes are isolated from biological body fluids, while plant-derived exosomes are isolated from apoplastic washing fluids. For mammalian-derived exosomes, current isolation and purification technologies mainly include microfluidic technology, ultracentrifugation method, exosome size-based separation technology, precipitation technology, and immunoaffinity capture technology, while available methods for isolating and purifying plant-derived exosomes are mainly based on established technologies for mammalian-derived exosomes, but have defects of different degrees. For example, for differential centrifugation, due to physiological differences between plants and animals, plant-derived exosomes obtained by this method are often mixed with proteins, nucleic acid aggregates, and other vesicles, and therefore further purification by density gradient ultracentrifugation is further required to isolate contaminants. For another example, due to the presence of high molecular weight components in plant juice, such as cellulose and starch, which usually cause difficulty in centrifugation, and therefore combinations of various centrifugation methods, for example a combination of differential centrifugation and sucrose density gradient centrifugation, need to be used to solve the above defects of conventional centrifugation technologies. In addition, although several other separation technologies, such as immunoaffinity capture, ultrafiltration or size-exclusion chromatography (SEC), co-precipitation methods, and microfluidic technology, have been successfully applied to mammalian-derived exosomes, they are not ideal for the isolation of plant-derived exosomes. For example, although immunoaffinity capture based on formation of immune complexes against extracellular vesicle surface antigens is an ideal method for purifying mammalian cell exosomes, for plant-derived exosomes, the insufficiency of marker proteins and characteristic antibodies relating to plant-derived exosomes limits the application of this technology. Therefore, methods suitable for extraction of mammalian exosomes are not suitable for extraction of plant exosomes.

The isolation of plant exosomes lacks a universal method, and currently commonly used in research are ultracentrifugation method, or a combination of multiple means such as extraction, membrane filtration for impurity removal, microfiltration for sterilization, nanofiltration concentration, and even assisted complexation reaction to obtain plant exosomes ^{[7]}. As described above, although ultracentrifugation method is generally considered the gold standard for exosome isolation and can isolate relatively pure exosomes ^{[5]}, the ultracentrifugation method has many defects and relies on ultrahigh-speed centrifugal force and duration, and achieves the purpose of isolation by sedimenting particles to the bottom, and therefore is time-consuming and the extraction scale is limited by the processing volume of the ultracentrifuge.

The skin is an important protective barrier against external stimuli such as bacteria and ultraviolet; the skin barrier is mainly consisted of the stratum corneum and keratinocytes, and its main function is to protect tissues and fluids in the body from the influence of the external environment. Irradiation by ultraviolet can cause damage to the stratum corneum of the skin and decreased expression of intracellular barrier proteins, thereby impairing the skin barrier function. At the same time, the skin is also a target organ of ultraviolet-induced oxidative damage, and the energy carried by UVB can activate endogenous photosensitive substances in the skin to generate various reactive oxygen species such as singlet oxygen, oxygen free radicals, and hydrogen peroxide. Therefore, inhibiting ultraviolet-induced intracellular reactive oxygen species response and disruption of the permeability barrier of the skin is one of the key elements for resisting skin photoaging and skin lesions.

Therefore, there is a need for a simple and efficient method suitable for large-scale isolation and extraction of high-purity plant exosomes at an industrial level, so as to achieve reduction of exosome production cost and improvement of yield, while the exosomes have beneficial effects on the skin.

### SUMMARY OF THE INVENTION

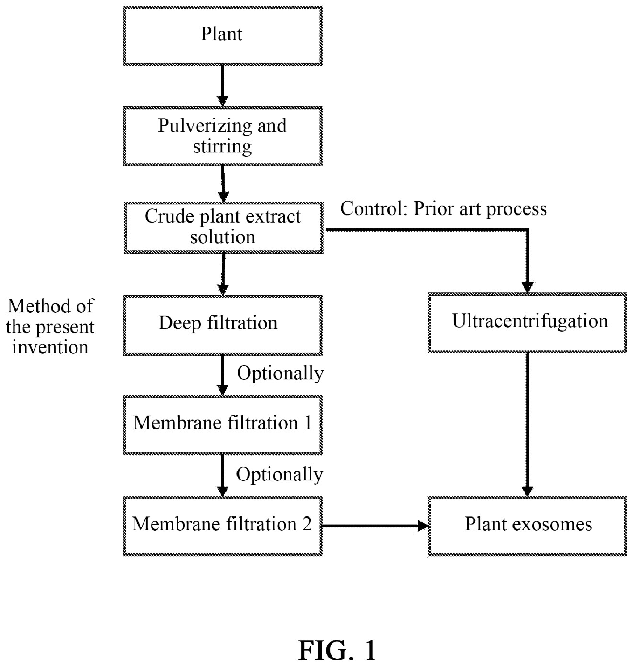

In order to overcome the deficiencies and shortcomings of plant exosome extraction methods in the prior art, the inventors for the first time propose a method for isolating and extracting plant exosomes using layer-by-layer filtration, which is simple, efficient, suitable for industrial scale (as shown in FIG. 1), and the isolated and purified exosomes have relatively high purity.

In this study, plant extracellular vesicles are obtained by extraction using a layer-by-layer filtration method, and through comparison it is found that the anti-aging effect of Dendrobium officinale extracellular vesicles (SH) is superior to that of other plants, and the anti-aging effect of Dendrobium officinale exosomes obtained by this extraction method is superior to that of Dendrobium officinale exosomes obtained by ultracentrifugation (UC) and Dendrobium officinale polysaccharides obtained by alcohol extraction (EE). We further elucidated the efficacy and mechanism of SH in anti-aging and firming through experiments.

Specifically, the present disclosure uses two methods, layer-by-layer filtration and ultracentrifugation, to isolate and obtain plant exosomes from Dendrobium (preferably Dendrobium officinale). By analyzing the physical characteristics and efficacy of the isolated plant exosomes and in combination with plant metabolomics analysis, differences between plant exosomes extracted by the two methods are compared, and thereby a method suitable for isolation and extraction of plant exosomes is screened.

The research results show that, compared with the ultracentrifugation method, the time for layer-by-layer filtration treatment of the same mass of plant raw material is shortened by 3 times, the yield is increased by at least 40 times, and metabolomics analysis shows that plant exosomes isolated and extracted by layer-by-layer filtration contain more active metabolites. When the plant raw material is scaled up by 10 times or even 20 times, the advantages of the method for isolating and extracting plant exosomes by layer-by-layer filtration are more obvious. In addition, plant exosomes isolated and extracted by layer-by-layer filtration have a typical exosome-like saucer shape, are rich in protein content, and have anti-inflammatory and immunoregulatory functional activities in vitro.

Specifically, compared with ultracentrifugation, the layer-by-layer filtration method of the present disclosure has lower time consumption, has higher exosome yield or particle concentration, can prevent filter membrane clogging caused by excessive impurities, and can ensure sterility of the product. Plant exosomes extracted using the layer-by-layer filtration method of the present disclosure have a typical exosome-like saucer shape, have a particle size within the range of 30-200 nm, and exhibit excellent anti-inflammatory and immunoregulatory activities.

The plant exosome extraction method of the present disclosure is suitable for large-scale production; preferably, the method can process an amount of plant material corresponding to large-scale production by increasing the membrane area for filtration, for example plant material with a mass of at least about 100 g, 200 g, 300 g, 400 g, 500 g, 600 g, 700 g, 800 g, 900 g, 1000 g, 2000 g, 3000 g, 4000 g, 5000 g, 6000 g, 7000 g, 8000 g, 9000 g, 10000 g, 50000 g, or even 100000 g.

Therefore, in one aspect, the present disclosure provides a method for isolating and extracting plant exosomes, specifically a method for isolating and extracting plant exosomes by deep filtration, the method comprises the following steps:
(a) obtaining a plant material;
(b) pretreating the obtained plant material to obtain a plant crude extract solution; and
(c) subjecting the obtained plant crude extract solution to layer-by-layer filtration treatment,
wherein in step (c), the layer-by-layer filtration treatment comprises a deep filtration step, and optionally a membrane filtration step.

In an embodiment of the method for isolating and extracting plant exosomes of the present disclosure, in step (c), the step of layer-by-layer filtration includes using deep filtration, preferably, the deep filtration employs deep filtration with a pore size of about 1-50 µm, more preferably deep filtration with a pore size of about 2-30 µm. In a specific embodiment, in step (c), the step of layer-by-layer filtration includes a deep filtration step, wherein the deep filtration step uses depth capsule filtration (preferably having a pore size of about 1-50 µm), more preferably the depth capsule filtration is depth capsule filtration having a pore size of about 2-30 µm.

In a specific embodiment, in step (c), the deep filtration step includes using a depth filter, preferably, the depth filter is a depth filter having a pore size of about 1-50 µm, more preferably a depth filter having a pore size of 2-30 µm. In a specific embodiment, the depth filter is a depth capsule filter having a pore size of 1-50 µm, more preferably a depth capsule filter having a pore size of about 2-30 µm, for example, suitable depth capsule filters include depth filters manufactured by Pall Corporation (NY), for example Supracap^{™} Depth Filter Capsules.

In an embodiment of the method for isolating and extracting plant exosomes of the present disclosure, the layer-by-layer filtration further includes at least one membrane filtration step performed after the deep filtration step, preferably, the membrane filtration includes anti-clogging membrane filtration, or further includes sterilizing membrane filtration, for example anti-clogging membrane filtration with a pore size of about 0.4-0.8 µm and/or sterilizing membrane filtration with a pore size of about 0.1-0.3 µm are performed, more preferably, the membrane filtration includes membrane filtration of about 0.45-0.8 µm and membrane filtration of about 0.1-0.22 µm, most preferably, the membrane filtration consists of sequentially performed anti-clogging membrane filtration of about 0.45 µm and sterilizing membrane filtration of about 0.22 µm.

In an embodiment of the method for isolating and extracting plant exosomes of the present disclosure, the flow rate of the deep filtration, membrane filtration of 0.45 µm, and membrane filtration of 0.22 µm is about 20-1000 mL/min, for example about 20-500 mL/min, about 20-200 mL/min, and about 30-100 mL/min.

In an embodiment of the method for isolating and extracting plant exosomes of the present disclosure, the layer-by-layer filtration of step (c) consists of deep filtration generally or specifically or preferably defined in the present disclosure and membrane filtration generally or specifically or preferably defined in the present disclosure.

In an embodiment of the method for isolating and extracting plant exosomes of the present disclosure, in step (b), the pretreatment includes one or more of mixing, pulverizing, grinding, stirring, filtering, and centrifuging, so as to obtain a plant crude extract. In a specific embodiment, the pretreatment includes mixing, pulverizing, filtering, and centrifuging.

In a specific embodiment, the mixing in step (b) uses an aqueous solution, for example an aqueous solution; preferably, the aqueous solution is an isotonic aqueous solution; more preferably, the isotonic aqueous solution is an isotonic sodium chloride aqueous solution, for example sodium chloride injection.

In a specific embodiment, the step (b) may be performed at any suitable temperature. For example, the step (b) may be performed at the following temperatures: about 0°C, 1°C, 2°C, 3°C, 4°C, 5°C, 6°C, 7°C, 8°C, 9°C, 10°C, 11°C, 12°C, 13°C, 14°C, 15°C, 16°C, 17°C, 18°C, 19°C, 20°C, 25°C, 30°C, 35°C, or 40°C, or any subrange within about 0°C to about 40°C (for example, any range between any two of the above temperatures), preferably about 2-30°C, more preferably 2-20°C, further preferably 2-8°C, and most preferably 4°C. In some embodiments, the step (b) of the present disclosure may be performed at ambient temperature (for example 25°C).

In an embodiment of the method of the present disclosure, the plant material may be terrestrial or aquatic. Preferably, the plant material is selected from Dendrobium (preferably Dendrobium officinale).

In another aspect, the present disclosure provides a plant exosome (preferably a Dendrobium exosome, more preferably a Dendrobium officinale exosome), which is prepared by the embodiment of the method generally or preferably defined above; preferably, the exosome has a diameter of about 30-450 nm, preferably a diameter of about 30-250 nm, more preferably a diameter of about 30-200 nm; and/or the exosome has a saucer shape; and/or compared with a plant exosome prepared by an ultracentrifugation method, the exosome has more metabolites in one or more of the following aspects: lipids and lipid-like molecule, organic oxygen compound, phenylpropanoids and polyketides, organicheterocyclic compound, organic acids and derivatives thereof, and/or benzenoid compound.

In another aspect, the present disclosure provides a plant exosome (preferably Dendrobium, more preferably Dendrobium officinale exosome) composition, for example an exosome solution, comprising plant exosomes prepared by the method generally or preferably defined in the present disclosure, and optionally a suitable excipient or carrier, wherein the excipient or carrier is acceptable in terms of nutrition and health care, pharmacy, food, dermatology, and/or cosmetics.

In a specific embodiment, the exosome composition is a pharmaceutical composition. In a specific embodiment, the exosome composition comprises Dendrobium (preferably Dendrobium officinale) exosomes prepared by the method of the present disclosure; and optionally a pharmaceutically acceptable carrier.

In a specific embodiment, the exosome composition is a food composition, which comprises plant exosomes prepared by the method generally or preferably defined in the present disclosure, and optionally other food ingredients and/or edible excipients and/or carriers. In a specific embodiment, the food composition comprises Dendrobium (preferably Dendrobium officinale) exosomes prepared by the method of the present disclosure, and optionally other food ingredients and/or edible excipients and/or carriers.

In a specific embodiment, the exosome composition is a beauty (or cosmetic) composition, which comprises plant exosomes prepared by the method generally or preferably defined in the present disclosure, and optionally a beauty/cosmetically acceptable excipient or carrier. In a specific embodiment, the beauty composition comprises Dendrobium (preferably Dendrobium officinale) exosomes prepared by the method of the present disclosure, and at least one beauty/cosmetically acceptable excipient or carrier.

In a specific embodiment, the exosome composition is a nutrition and health-care composition, which comprises plant exosomes prepared by the method generally or preferably defined in the present disclosure, and optionally a nutrition and health-care acceptable excipient or carrier. In a specific embodiment, the nutrition and health-care composition comprises Dendrobium (preferably Dendrobium officinale) exosomes prepared by the method of the present disclosure, and at least one nutrition and health-care acceptable excipient or carrier.

In another aspect, the present disclosure further provides use of plant exosomes (preferably Dendrobium, more preferably Dendrobium officinale exosomes) prepared by the method of the present disclosure or a composition comprising the same (including but not limited to a pharmaceutical composition/food composition/beauty or cosmetic composition/nutrition and health-care composition), for treating or preventing-or helping (or assisting in) improving, alleviating, or controlling-inflammation, preferably skin inflammation, or for use in the dermatological field-or assisting in-repairing, anti-inflammation, antioxidation, anti-aging, whitening, or moisturizing.

In another aspect, the present disclosure further provides use of plant exosomes (preferably Dendrobium, more preferably Dendrobium officinale exosomes) prepared by the method of the present disclosure or a composition comprising the same (including but not limited to a pharmaceutical composition/food composition/beauty composition/nutrition and health-care composition) in preparation of a product (drug, food, nutrition and health-care product, beauty/cosmetic product, and the like), wherein the product is used for treating or preventing-or helping(or assisting in) improving, alleviating, or controlling-inflammation, preferably skin inflammation, or for use in the dermatological field or assisting in repairing, anti-inflammation, antioxidation, anti-aging, whitening, or moisturizing.

In another aspect, the present disclosure provides a method for treating or preventing inflammation, preferably skin inflammation, or treating or preventing dermatological symptoms, or assisting in improving, alleviating, or controlling dermatological symptoms, comprising administering to a subject in need thereof plant exosomes (preferably Dendrobium, more preferably Dendrobium officinale exosomes) prepared by the method generally or preferably defined above or a composition comprising the same (pharmaceutical composition/food composition/beauty or cosmetic composition/nutrition and health-care composition).

In another aspect, the present disclosure provides use of the above plant exosomes (preferably Dendrobium, more preferably Dendrobium officinale exosomes) or plant exosome composition in biomedicine or a drug delivery system.

In another aspect, the present disclosure further provides a method for preparing a drug, food, nutrition and health-care product, or beauty/cosmetic composition, comprising preparing plant exosomes (preferably Dendrobium, more preferably Dendrobium officinale exosomes) according to the method generally or preferably defined in the present disclosure, and incorporating the plant exosomes into the drug, food, nutrition and health care-product, or beauty/cosmetic composition.

### DESCRIPTION OF DRAWINGS

The following drawings illustrate preferred embodiments of the present disclosure. For the purpose of illustrating the present disclosure, currently preferred embodiments are shown in the drawings. However, it should be understood that the present disclosure is not limited to the specific embodiments shown in the drawings.
FIG. 1 shows an exemplary step flow of the plant exosome isolation and extraction method of the present disclosure.

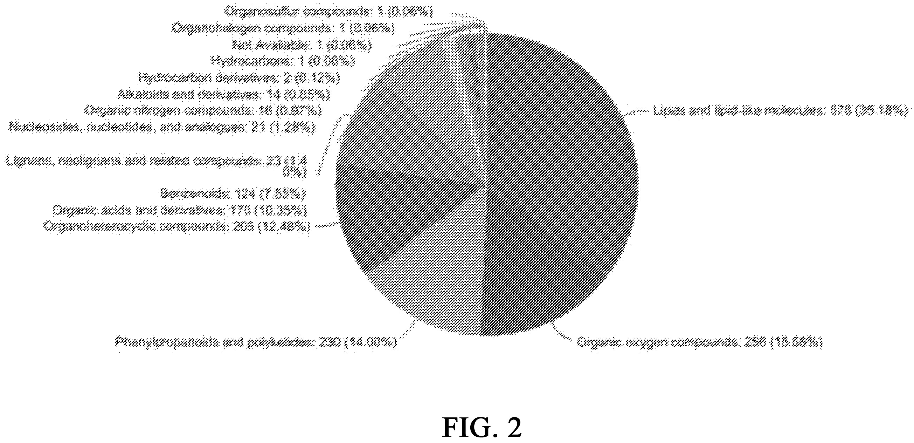

FIG. 2 shows compound classification SHV01 of Dendrobium officinale exosomes extracted by the layer-by-layer filtration method of the present disclosure.

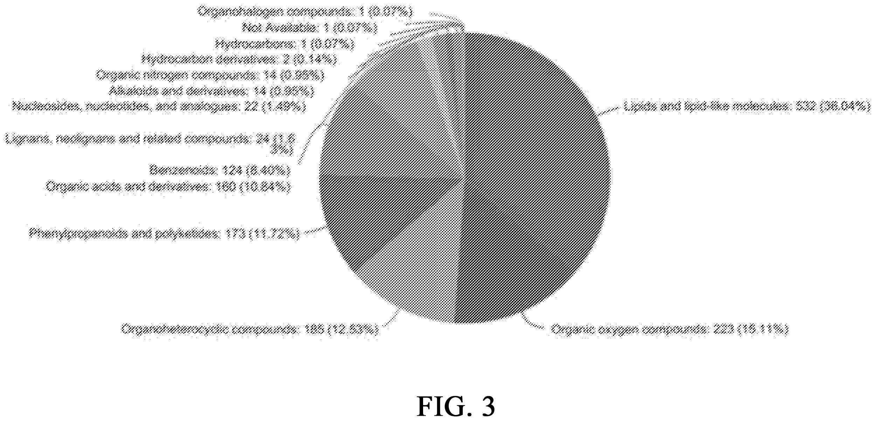

FIG. 3 shows compound classification DeE01 of Dendrobium officinale exosomes extracted by an ultracentrifugation method.

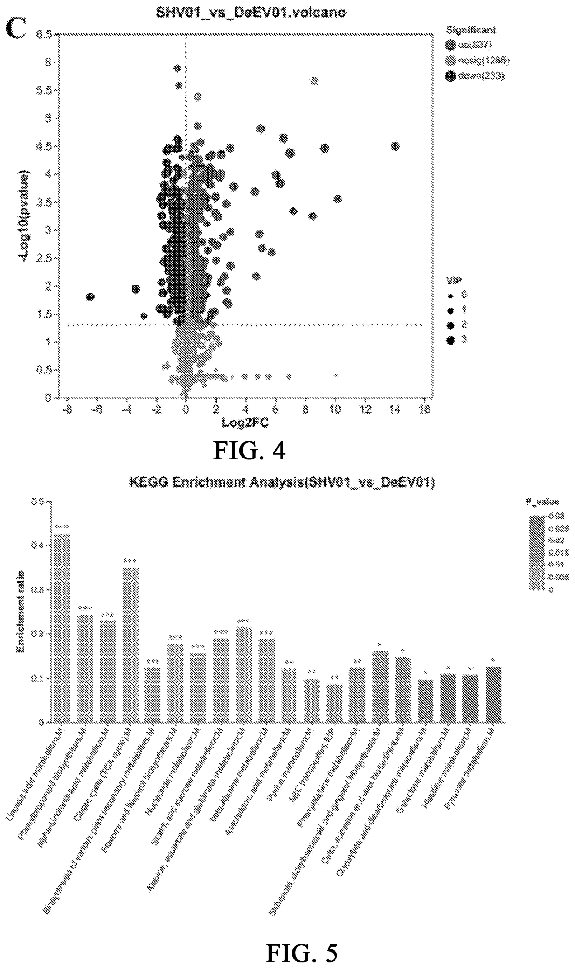

FIG. 4 shows a volcano plot of differential metabolites of Dendrobium officinale exosomes extracted using layer-by-layer filtration and ultracentrifugation: SHV01 vs DeE01. The abscissa is the fold change value of expression differences of metabolites between the two groups, namely log2FC; the ordinate is the statistical test value of differences in changes of metabolite expression levels, namely the -log10(p value) value, and the values of both the abscissa and the ordinate have been logarithmically processed.
FIG. 5 shows a KEGG pathway enrichment analysis diagram of Dendrobium officinale exosomes extracted using layer-by-layer filtration and ultracentrifugation: SHV01 vs DeE01. The abscissa represents pathway names, and the ordinate represents enrichment rate, indicating the ratio of the number of metabolites enriched in the pathway (Metabolite number) to the number of metabolites annotated to the pathway (Background number); the larger the ratio, the higher the degree of enrichment. The color gradient of the columns indicates the significance of enrichment; by default, the darker the color, the more significantly enriched the KEGG term, wherein P values < 0.001 are marked as ***, P values < 0.01 are marked as **, and P values < 0.05 are marked as *.

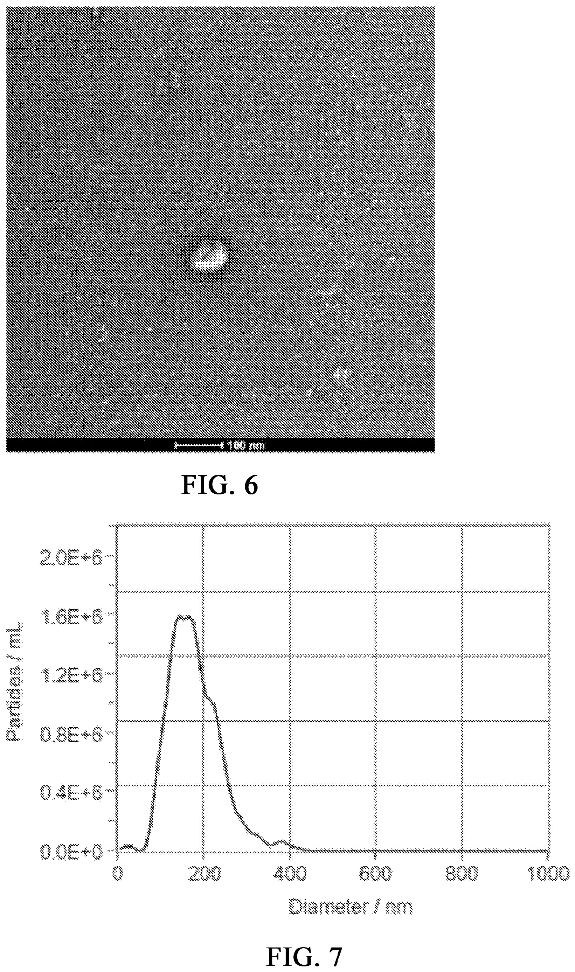

FIG. 6 shows a TEM analysis image SHV01 of Dendrobium officinale exosomes isolated and extracted by layer-by-layer filtration.
FIG. 7 shows the particle size and concentration of SHV01 by nanoparticle tracking analysis.

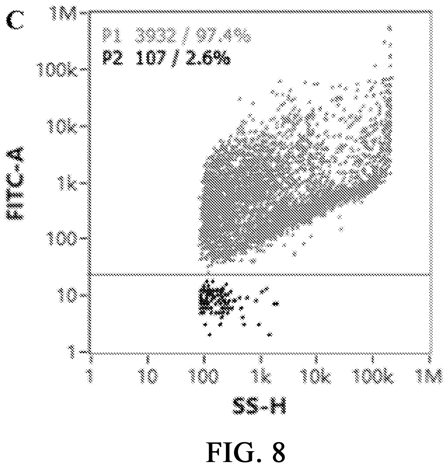

FIG. 8 shows in vitro PKH67 staining flow detection SHV01 of Dendrobium officinale exosomes isolated and extracted by layer-by-layer filtration.

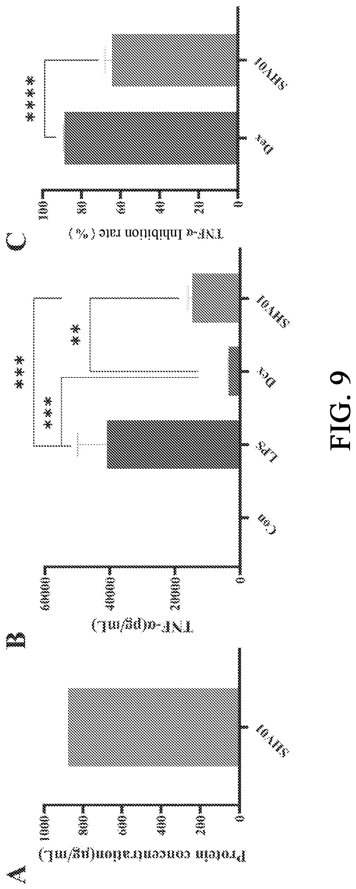

FIG. 9 shows activity detection of Dendrobium officinale exosomes extracted by layer-by-layer filtration; A: protein concentration of Dendrobium officinale exosomes. B: Dendrobium officinale exosomes inhibit LPS-induced release of TNF-α by RAW264.7. The ordinate is the concentration of TNF-α detected by an ELISA kit. P values < 0.001 are marked as ***; C: TNF-α release inhibition rate of Dendrobium officinale exosomes.

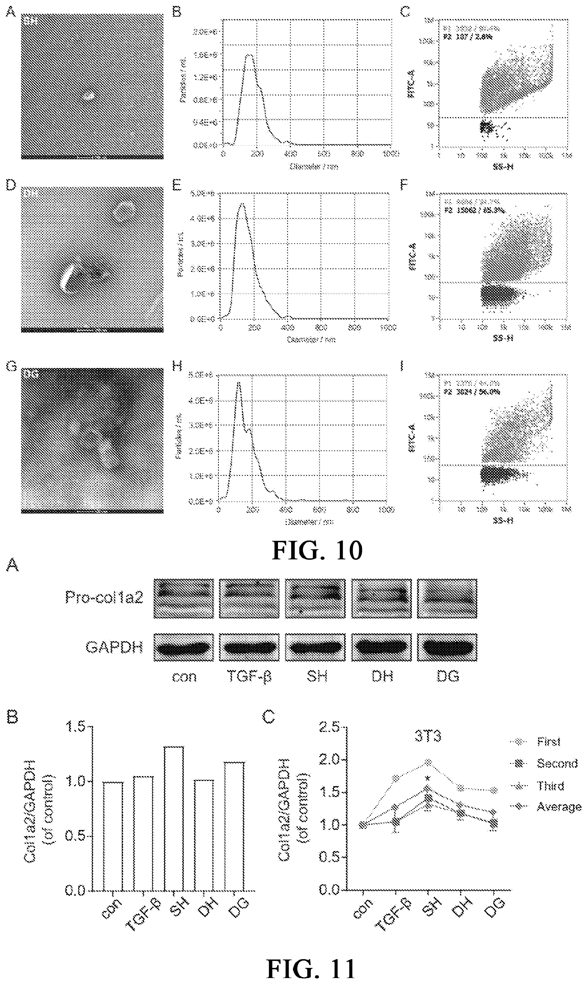

FIG. 10 shows physical characterization of different plant extracellular vesicles. A. TEM analysis of the shape of Dendrobium officinale extracellular vesicles (SH); B. nanoparticle tracking analysis of the particle size and concentration of Dendrobium officinale extracellular vesicles; C. nano flow cytometry analysis of the PKH67 positive rate of Dendrobium officinale extracellular vesicles; D. TEM analysis of the shape of Rehmannia glutinosa extracellular vesicles (DH); E. nanoparticle tracking analysis of the particle size and concentration of Rehmannia glutinosa extracellular vesicles; F. nano flow cytometry analysis of the PKH67 positive rate of Rehmannia glutinosa extracellular vesicles; G. TEM analysis of the shape of Angelica sinensis extracellular vesicles (DG); H. nanoparticle tracking analysis of the particle size and concentration of Angelica sinensis extracellular vesicles; I. nano flow cytometry analysis of the PKH67 positive rate of Angelica sinensis extracellular vesicles.
FIG. 11 shows the promoting effect of different plant extracellular vesicles on the basal collagen level of fibroblasts. A. WB detects differences in the level of type I collagen expressed by 3T3 cells. The concentrations of the extracellular vesicle treatment groups are all 1×10⁸ particles/mL, and the specific treatment groups and corresponding mixed plants have been marked in the figure (con: blank control group; TGF-β: positive control group; SH: Dendrobium officinale; DH: Rehmannia glutinosa; DG: Angelica sinensis); B. semi-quantitative analysis of the WB results in panel A; C. statistical difference analysis of the results of three repeated experiments is performed using a two-tailed Student's T test, and the mean and SD values are marked in red. *: P-value < 0.05.

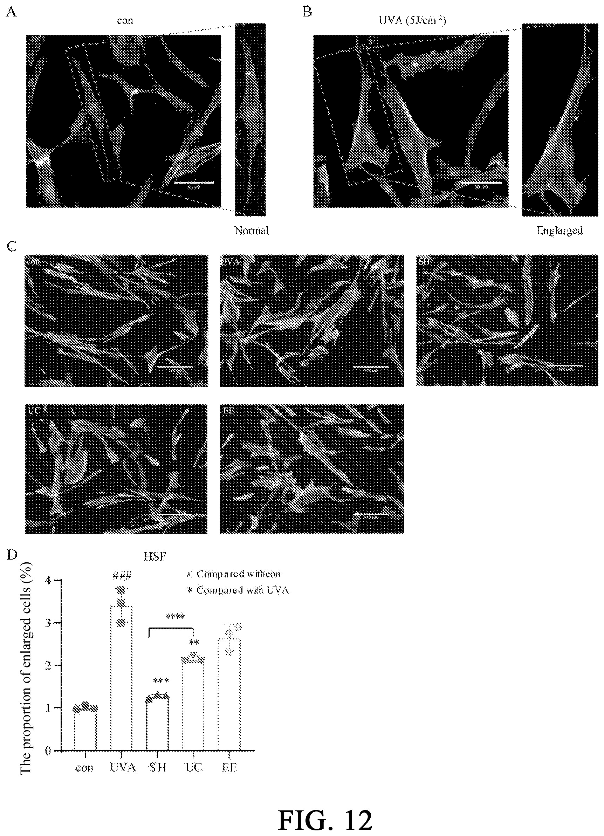

FIG. 12 shows comparison of functional components of Dendrobium officinale obtained by different extraction processes in improving UVA-induced cell enlargement. A. Normal cells are imaged after fluorescent labeling with phalloidin, and representative cell morphology is listed on the right; B. after HSF modeling by stimulation with 5 J/cm² UVA, representative images of enlarged cells are listed on the right; C. HSF cells fluorescently labeled with phalloidin under a fluorescence microscope. The concentrations of the SH and UC treatment groups are both 1×10⁸ particles/mL, and the concentration of the EE treatment group is 0.025% (v/v); the specific treatment groups have been marked in the figure (con: blank control group; UVA: ultraviolet irradiation group; SH: layer-by-layer filtration; UC: ultracentrifugation; EE: alcohol extraction); D. quantitative analysis of the fluorescence imaging results in panel A, wherein difference analysis is performed using a two-tailed Student's T test, # indicates the difference between the data group and the con group, ###: P-value < 0.001, * indicates the difference from the UVA group, **: P-value < 0.01, ***: P-value < 0.001, and ****: P-value < 0.0001.

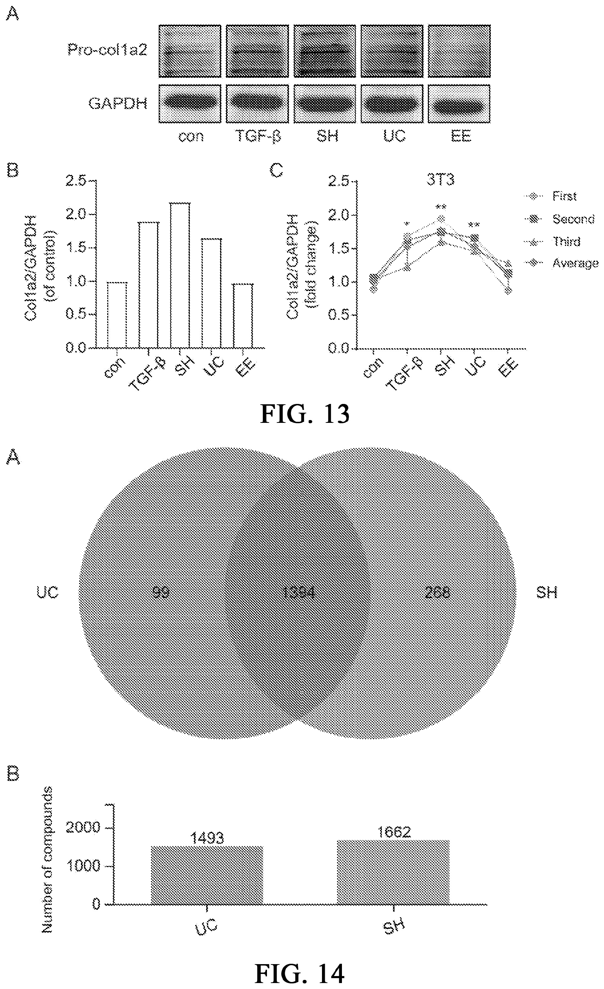

FIG. 13 shows the promoting effect of functional components of Dendrobium officinale obtained by different extraction processes on the basal collagen level of fibroblasts. A. WB detects differences in the level of type I collagen expressed by 3T3 cells. The concentrations of the SH and UC treatment groups are both 1×10⁸ particles/mL, and the concentration of the EE treatment group is 0.025% (v/v); the specific treatment groups have been marked in the figure (con: blank control group; UVA: ultraviolet irradiation group; SH: layer-by-layer filtration; UC: ultracentrifugation; EE: alcohol extraction); B. semi-quantitative analysis of the WB results in panel A; C. statistical difference analysis of the results of three repeated experiments is performed using a two-tailed Student's T test, and the mean and SD values are marked in red. *: P-value < 0.05. After folding, COLI protein is a heterotrimer composed of two α1 chains and one α2 chain. The antibody used in the experiments of the present disclosure during WB specifically recognizes the pro-α2 chain of type I collagen, which is represented by COL1a2.
FIG. 14 shows Venn diagram analysis of Dendrobium officinale extracellular vesicles obtained by different isolation methods. A. Different colors in the figure represent differential metabolites of different comparison groups, the overlapping parts represent the number of metabolites shared by multiple metabolite sets, the non-overlapping parts represent the number of metabolites unique to the metabolite set, and the numbers represent the corresponding numbers of metabolites; B. the bar chart represents the number of metabolites comprised in each metabolite set.

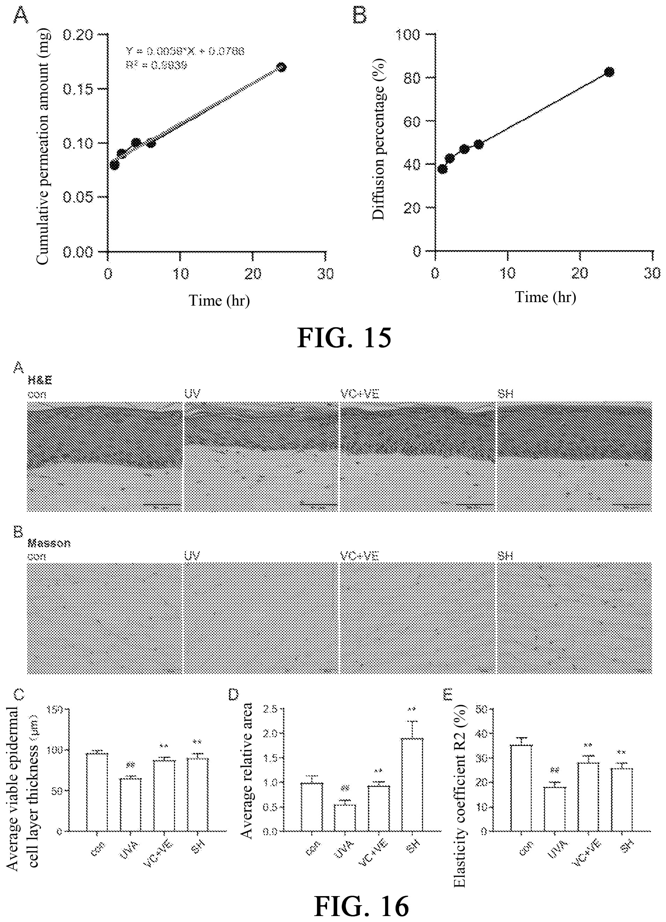

FIG. 15 shows a transdermal study of SH. A. Cumulative permeation amount of samples of SH at different time points and fitting of the regression equation (the linear fit is marked in red); B. diffusion percentage of samples of SH at different time points.
FIG. 16 shows the anti-aging effect of SH on a UV-irradiated 3D skin model. A. Representative tissue sections (H&E staining) of tissue morphology of the 3D skin model, with each treatment group marked in the figure (con: blank control group; UV: negative control; VC+VE: positive control, with treatment concentrations of VC: 100 µg/mL and VE: 7 µg/mL; SH: Dendrobium officinale extracellular vesicles obtained by layer-by-layer filtration, with a treatment total amount of 4×10⁸ particles); B. representative sections of tissue collagen fibers (Masson staining); C. quantitative analysis of tissue morphology results; D. quantitative analysis of collagen fiber results; E. tissue elasticity coefficient R2. When statistical analysis is performed by a t-test method, # indicates the difference between the data group and the con (control) group, ##: P-value < 0.01, * indicates the difference from the UVA group, and **: P-value < 0.01.

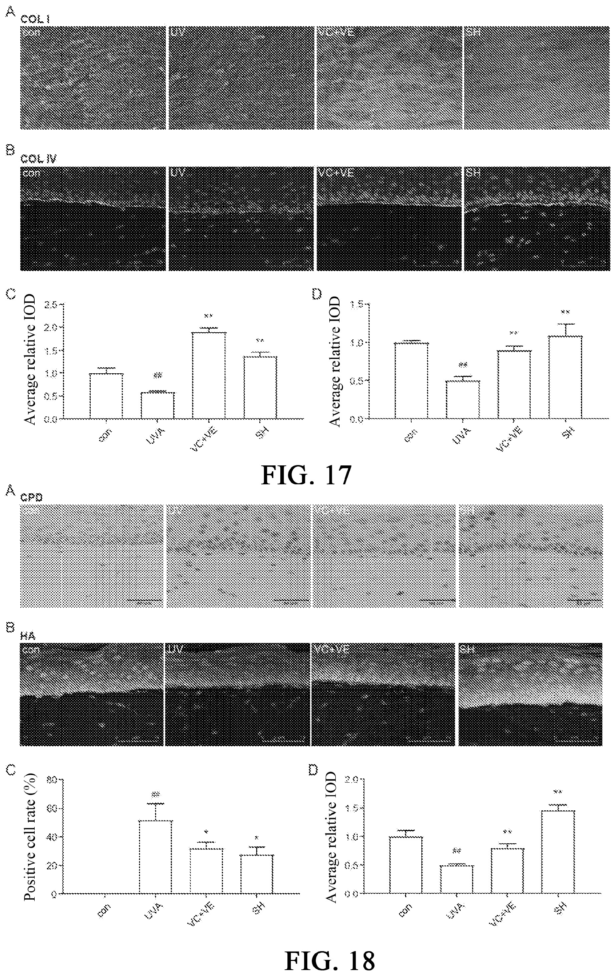

FIG. 17 shows the effect of SH on collagen generation in a UV-irradiated 3D skin model. A. Representative immunofluorescence image of type I collagen (COL I); B. representative immunofluorescence image of type IV collagen (COL IV); C. quantitative analysis of the relative integrated optical density (IOD) value of COL I; D. quantitative analysis of the relative integrated optical density (IOD) value of COL IV. VC+VE is the positive control, with treatment concentrations of VC: 100 µg/mL and VE: 7 µg/mL; SH represents Dendrobium officinale extracellular vesicles obtained by layer-by-layer filtration, with a treatment total amount of 4×10⁸ particles. When statistical analysis is performed by a t-test method, # indicates the difference between the data group and the con (control) group, ##: P-value < 0.01, * indicates the difference from the UV group, and **: P-value < 0.01.
FIG. 18 shows the effect of SH on expression of cyclobutane-pyrimidone dimer and hyaluronic acid in a UV-irradiated 3D skin model. A. Representative immunohistochemical image of cyclobutane-pyrimidone dimer (CPD); B. representative immunofluorescence image of hyaluronic acid (HA); C. statistical results of CPD-positive cell rate; D. quantitative analysis of the relative integrated optical density (IOD) value of HA. VC+VE is the positive control, with treatment concentrations of VC: 100 µg/mL and VE: 7 µg/mL; SH represents Dendrobium officinale extracellular vesicles obtained by layer-by-layer filtration, with a treatment total amount of 4×10⁸ particles. When statistical analysis is performed by a t-test method, # indicates the difference between the data group and the con (control) group, ##: P-value < 0.01, * indicates the difference from the UV group, *: P-value < 0.05 and **: P-value < 0.01.

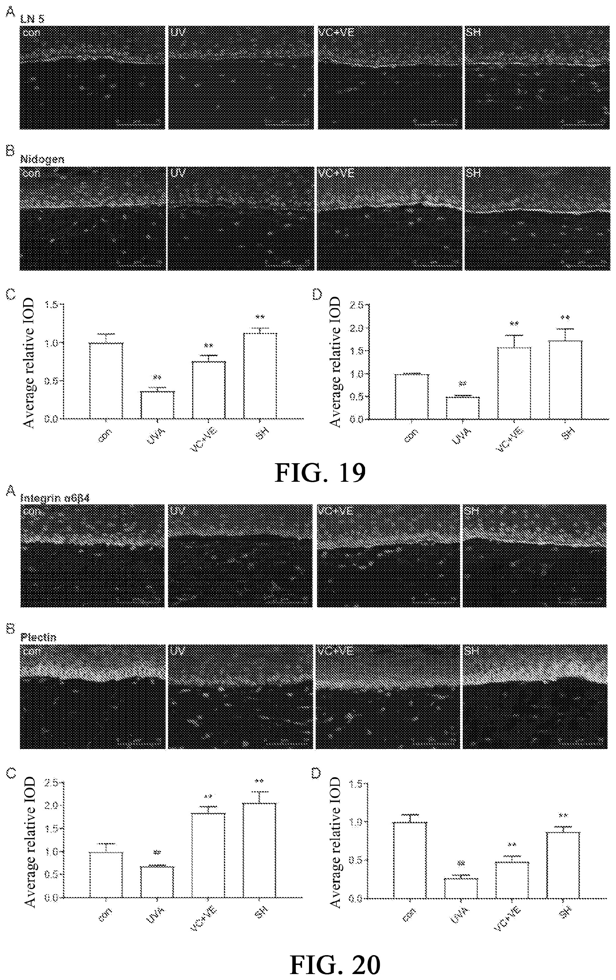

FIG. 19 shows the effect of SH on basement membrane structural proteins in a UV-irradiated 3D skin model. A. Representative immunofluorescence image of laminin 5 (LN5); B. representative immunofluorescence image of nidogen; C. quantitative analysis of the relative integrated optical density (IOD) value of LN5; D. quantitative analysis of the relative integrated optical density (IOD) value of Nidogen. VC+VE is the positive control, with treatment concentrations of VC: 100 µg/mL and VE: 7 µg/mL; SH represents Dendrobium officinale extracellular vesicles obtained by layer-by-layer filtration, with a treatment total amount of 4×10⁸ particles. When statistical analysis is performed by a t-test method, # indicates the difference between the data group and the con (control) group, ##: P-value < 0.01, * indicates the difference from the UV group, and **: P-value < 0.01.
FIG. 20 shows the effect of SH on junction proteins in a UV-irradiated 3D skin model. A. Representative immunofluorescence image of integrin α6β4; B. representative immunofluorescence image of plectin; C. quantitative analysis of the relative integrated optical density (IOD) value of Integrin α6β4; D. quantitative analysis of the relative integrated optical density (IOD) value of Plectin. VC+VE is the positive control, with treatment concentrations of VC: 100 µg/mL and VE: 7 µg/mL; SH represents Dendrobium officinale extracellular vesicles obtained by layer-by-layer filtration, with a treatment total amount of 4×10⁸ particles. When statistical analysis is performed by a t-test method, # indicates the difference between the data group and the con (control) group, ##: P-value < 0.01, * indicates the difference from the UV group, and **: P-value < 0.01.

### DETAILED DESCRIPTION OF THE INVENTION

### I. Definitions and terms

Unless otherwise specified, all technical and scientific terms used herein have the same meanings as commonly understood by a person of ordinary skill in the art to which the present disclosure belongs.

As used in the context of the present disclosure, a "vesicle," also referred to as an extracellular vesicle, is a membrane-enclosed structure that is released by cells in vitro and in vivo. Extracellular vesicles may contain proteins, lipids, and nucleic acids, and may mediate intercellular communication among different cells (including different cell types) in vivo. Two types of extracellular vesicles are exosomes and microvesicles.

As used in the context herein, "exosomes" refer to lipid-bound, cell-secreted small vesicles that are released from cells by fusion of multivesicular endosomes (MVEs) with the plasma membrane and mediate intercellular communication through intercellular transport of proteins and RNA. Usually, the size range of exosomes is from about 30 nm to about 200 nm, and sometimes may also be 30-150 nm or 30-100 nm, and they have a bilayer membrane structure and saucer-shaped morphology.

As used in the context herein, "plant material" has the meaning commonly understood in the art. The plant raw material may be in a fresh state or may be dried. Usually, except for individual plants (for example, Lycium barbarum), producing plant exosomes from fresh plant material is more preferred.

As used in the context herein, "deep filtration (DF)" means removing particles (for example impurities) from a liquid such as a solution using a deep filtration medium, wherein particles and impurities are retained throughout the depth portion of the porous structure of the filtration medium by a combination of size (sieving/filtration) and intermolecular interactions (for example electrostatic attraction and hydrophobic interaction between oppositely charged surfaces). Deep filtration has been successfully used for primary clarification of bacterial, yeast, insect, and mammalian cell suspensions, and may be used alone or in combination with centrifugation. The deep filtration medium may comprise cellulose, for example cellulose fibers, and/or polypropylene, for example polypropylene fibers, and/or filter aids (such as activated carbon, diatomaceous earth (DE), and/or perlite), and/or resin (for example polymeric resin). In some embodiments, medium-sized particles are removed less by deep filtration; very small particles are effectively removed by Brownian diffusion (plus adsorption) (for example, particles smaller than the voids of the medium can enter into the interior of the medium), while very large particles are captured by physical sieving or interception (for example, retained and attached on the medium).

As used herein, the term "depth filter " or "depth capsule filter" means filtration is achieved within the depth of the filter material. Such filters are filters that comprise a random fiber matrix combined to form a complex, tortuous maze of flow channels. Particle isolation in these filters is caused by entrapment by or adsorption onto the fiber matrix. The most frequently used depth filter media for bioprocesses for cell culture broths and other feeds include cellulose fibers, filter aids such as DE, and positively charged resin binders. Unlike absolute filters, the depth filter medium retains particles throughout the porous medium, thereby allowing retention of particles larger than the pore size. Companies currently having deep filtration medium products in the industry include Sartorius, Merck Millipore, Pall (PALL), 3M, and the like. Commercially available depth filters include, but are not limited to, Millistak+Pod depth filter systems, XOHC medium (Millipore), Zeta Plus ^{™} depth filters (3M Purification), and the like. In the present disclosure, deep filtration may be performed using two or more depth filters arranged in parallel. In this case, commercially available depth filters may include, for example, Millistak+mini DOHC (Millipore) and XOHC filters (Millipore) or filters from Pall Corporation (NY), such as Supracap^{™} Depth Filter Capsules.

In the context herein , the terms "cosmetic," "cosmetic composition," "beauty product," or "beauty composition" refer to a chemical industrial product or fine chemical product that is spread on any part of the surface of a human body, such as skin, hair, fingernails and toenails, lips and teeth, by smearing, spraying, or other similar methods, for the purpose of cleaning, care, beautification, modification and changing appearance, or correcting human body odor and maintaining a good condition. In some embodiments, the cosmetic, cosmetic composition, beauty product, or beauty composition of the present disclosure is for non-therapeutic purposes.

In the context herein, the term "nutrition and health-care composition" refers to a product for supplementing the body's nutrient requirements and improving the body's health status and bodily functions, which may be administered gastrointestinally or parenterally, and is usually administered gastrointestinally.

In the context herein, "skin" should be understood as including layers thereof, from the uppermost layer or stratum corneum to the lowermost layer or subcutaneous layer (including both the uppermost layer or stratum corneum and the lowermost layer or subcutaneous layer). These layers are composed of different types of cells, such as keratinocytes, fibroblasts, melanocytes, mast cells, neurons, and/or adipocytes, and the like. The term "skin" also includes scalp. The term "skin" comprises mammalian skin and comprises human skin. Similarly, the terms "hair, nails, and mucosa" comprise hair, nails, and mucosa of mammals (for example humans).

In the context herein, the term "treatment" encompasses therapeutic methods, comprising methods involving administration of an extract according to the present disclosure to reduce or eliminate a disease or disorder or to reduce or eliminate one or more symptoms associated with said disease or disorder. The term "treatment" also encompasses therapeutic methods involving reduction or elimination of physiological consequences of a disease or disorder.

In the context herein, the term "care" refers to maintaining characteristics of skin, hair, nails, and/or mucosa. Said characteristics are improved or maintained by cosmetic treatment and/or care in healthy subjects, in subjects having sensitive skin, and in subjects exhibiting symptoms of skin, hair, nails, and/or mucosa (for example, but not limited to, ulcers and skin injuries, psoriasis, dermatitis, acne, rosacea, and the like).

In the context herein, when the terms "treatment" and "care" are modified by "cosmetic" and/or "non-therapeutic," it means that the purpose of said treatment or care is to help or assist in improving, alleviating, or controlling the condition involved, for example to help or assist in improving or maintaining cosmetic characteristics of skin, hair, nails, and/or mucosa, which characteristics affect the aesthetic appearance of skin, hair, nails, and/or mucosa, specifically, for example, to help or assist in repairing, anti-inflammation, antioxidation, anti-aging, whitening, or moisturizing, and the like, so as to improve hydration, elasticity, firmness, gloss, tone, or texture of skin, hair, nails, and/or mucosa.

In the context herein, the term "prevention" refers to the ability of an extract of the present disclosure to prevent, delay, or impede the occurrence or development of a disease or disorder, or to prevent, delay, or impede changes in cosmetic characteristics of skin, mucosa, and/or hair. As used in the present disclosure, the term "prevention" may be used interchangeably with the term "inhibition of occurrence," that is, it refers to the ability of an extract of the present disclosure to inhibit the occurrence or development of a disease or disorder, or to inhibit changes in cosmetic characteristics of skin, hair, nails, and/or mucosa.

In the context herein, when used with respect to a particular numerical value or range of values, the term "about" means that the numerical value with which it is used varies by ±10%, for example by ±5%, ±2%, or ±1%. For example, as used herein, the expression "about 100" includes 90 and 110 and all values therebetween (for example 90.5, 95, 101, 105, 109.95, and the like). For ratios, the term "about" is used to define each number of the given ratio, for example, the ratio "about 1:1" refers to a ratio of (0.9-1.1): (0.9-1.1); and, for another example, the range "about n-m" or "about n-about m" refers to 90% n-110% n to 90% m-110% m.

Any methods and materials similar or equivalent to those methods and materials described herein may be used in the practice or testing of the present disclosure, and the methods and materials described below are merely exemplary.

All patents, applications, and non-patent publications mentioned in this specification are incorporated herein by reference in their entirety.

### II. Preparation of exosomes

### Raw material

The raw material involved in the method of the present disclosure is plant material. Preferably, the plant material may be terrestrial. More preferably, the plant material is Dendrobium (preferably Dendrobium officinale).

The plant material may be part or all of a plant, and for example may be selected from wood, roots, rhizomes, bark, trunks, flowers, petals, sepals, seeds, fruits, stems, leaves, and/or embryos, and mixtures of one or more thereof.

In some embodiments, the plant material is or includes Dendrobium Lindl.). Dendrobium that may be used in the method of the present disclosure may be dried Dendrobium or fresh Dendrobium, for example fresh or dried Dendrobium stems, or processed traditional Chinese medicine Dendrobium, such as processed stems of Dendrobium officinale. The Dendrobium that may be used in the method of the present disclosure for example but is not limited to: all or part of Dendrobium officinale (Dendrobium officinale), Dendrobium nobile, Dendrobium moniliforme, Dendrobium loddigesii, Dendrobium chrysotoxum, Dendrobium fimbriatum, Dendrobium chrysanthum, Dried stem of Dendrobium loddigesii, and Dendrobium phalaenopsis, or fresh stems thereof, or a mixture of one or more thereof. The method of the present disclosure may use a mixture of one or more Dendrobium species as the plant material for extraction of exosomes.

### Pretreatment

In some embodiments, the extraction method comprises pretreating plant material (preferably Dendrobium, more preferably Dendrobium officinale). Pretreatment techniques are well known in the art and include physical, chemical, and biological pretreatment, or any combination thereof.

In some embodiments, the pretreatment includes, but is not limited to, one or more of washing, mixing with an isotonic solution, pulverizing, grinding, stirring, filtering, and centrifuging. Preferably, the pretreatment is performed in a sterile environment.

In some embodiments, washing in the pretreatment may be performed with an aqueous carrier or another edible or pharmaceutically acceptable solvent (including an organic solvent or an inorganic solvent). Preferably, the washing agent is an aqueous carrier. Aqueous carriers are known in the art and include, but are not limited to, sterile water, water for injection, or isotonic solutions. Optionally, the washing treatment further comprises a certain amount of surfactant in the washing solvent. More preferably, the washing agent is water or water for injection.

In some embodiments, the isotonic solution used in the pretreatment includes a solution of an isotonic agent. Isotonic solutions include, but are not limited to, sodium chloride solution, phosphate buffer, Ringer's injection, isotonic glucose injection, and Ringer's injection of glucose and lactate. Isotonic agents include isotonic agents selected from sodium chloride, mannitol, lactose, glucose (hydrous or anhydrous), sucrose, glycerol, and sorbitol, or a solution of any one of the foregoing. In certain embodiments, sodium chloride is present in an isotonic amount. Preferably, the isotonic solution is a sterile solution containing 9 mg/ml (or 0.9%) sodium chloride or sodium chloride injection.

In some embodiments, plant material (preferably Dendrobium, more preferably Dendrobium officinale) is mixed with an isotonic solution at a certain ratio. For example, the plant material is mixed with the isotonic solution according to the following mass-to-volume ratios (w/v, g/mL, plant mass:isotonic solution volume): about 1:1, about 1:2, about 1:3, about 1:4, about 1:5, about 1:6, about 1:7, about 1:8, about 1:9, about 1:10, about 1:11, about 1:12, about 1:13, about 1:14, about 1:15, about 1:20, about 1:30, about 1:50, about 1:100, about 1:200, or about 1:500. Preferably, the range of the mass-to-volume ratio of plant material to isotonic solution is about 1:2-1:50, about 1:3-1:20, or about 1:5-1:20.

In some embodiments, pulverizing in the pretreatment includes any suitable manner known in the art, including but not limited to dry milling, wet milling, and vibratory ball milling. For example, pulverizing includes subjecting plants or parts thereof to pulverization treatment using a powdering technique or a cell wall disruption technology. The cell wall disruption technology uses a cell wall breaker.

In some embodiments, the pretreatment may be performed at any suitable temperature. For example, the pretreatment may be performed at the following temperatures: about 0°C, 1°C, 2°C, 3°C, 4°C, 5°C, 6°C, 7°C, 8°C, 9°C, 10°C, 11°C, 12°C, 13°C, 14°C, 15°C, 16°C, 17°C, 18°C, 19°C, 20°C, 25°C, 30°C, 35°C, or 40°C, or any sub-range within about 0°C to about 40°C (for example, any range between any two of the above temperatures), preferably about 2-30°C, more preferably about 2-20°C, further preferably about 2-8°C, and most preferably about 4°C. In some embodiments, the step (b) of the present disclosure may be performed at ambient temperature (for example about 25°C).

In some embodiments, the stirring, filtering, or centrifuging operation in the pretreatment may be selected and adjusted by a person skilled in the art according to specific needs.

### Layer-by-layer filtration

The plant exosome extraction method of the present disclosure comprises a step of layer-by-layer filtration.

The layer-by-layer filtration of the method of the present disclosure includes at least one filtration step, preferably multiple filtration steps, for example at least 2, 3, 4, 5, 6, 7, 8, 9, 10, or even more filtration steps. In some embodiments, for example, layer-by-layer filtration may include 3 filtration steps. In some embodiments, the 3 filtration steps may be performed independently or continuously. In some embodiments, the step of layer-by-layer filtration includes a deep filtration step and anti-clogging filtration; preferably, the anti-clogging filtration may include membrane filtration, for example 0.4-0.8 µm membrane filtration; more preferably, the step of layer-by-layer filtration includes a deep filtration step, anti-clogging filtration, and sterilizing filtration, preferably the anti-clogging filtration may include 0.4-0.8 µm membrane filtration and the sterilizing filtration includes 0.1-0.3 µm membrane filtration.

The method of the present disclosure may be used to process said plant (preferably Dendrobium, more preferably Dendrobium officinale) at a suitable rate. For example, for one production batch, the rate at which the method of the present disclosure processes plants ranges from about 30 g/hour, 50 g/hour, 60 g/hour, 70 g/hour, 80 g/hour, 90 g/hour, 100 g/hour, 200 g/hour, 300 g/hour, 400 g/hour, 500 g/hour, 600 g/hour, 700 g/hour, 800 g/hour, 900 g/hour, 1,000 g/hour, or any sub-range within about 10 g/hour to about 1,000 g/hour (for example, any range between any two of the above production rates).

The respective filtration steps of the layer-by-layer filtration of the method of the present disclosure may be the same or may be different. For example, filtration pore sizes between different filtration steps are different. In some embodiments, layer-by-layer filtration includes 1 filtration step, wherein the filtration step uses two filtration units with different pore sizes, preferably the two pore sizes become smaller sequentially along the fluid direction of the filtrate, for example the first filtration pore size is about 0.45 µm, and the second filtration pore size is about 0.22 µm. In some embodiments, layer-by-layer filtration includes 2 filtration steps, wherein the pore size in the first filtration step is different from the pore size in the second filtration step; for example, the two pore sizes become smaller sequentially along the fluid direction of the filtrate, for example the pore size in the first filtration step is about 0.45 µm, and the pore size in the second filtration step is about 0.22 µm; and for another example, the pore size in the first filtration step is about 2-30 µm (such as 30 µm), and the pore size in the second filtration step is about 0.45 µm. In some embodiments, layer-by-layer filtration includes 3 filtration steps, wherein the pore size in the first filtration step, the pore size in the second filtration step, and the pore size in the third filtration step are not completely the same or are at least partially different; preferably, the three pore sizes become smaller sequentially along the fluid direction of the filtrate, for example the pore size in the first filtration step is about 2-30 µm, the pore size in the second filtration step is about 0.45 µm, and the pore size in the third filtration step is about 0.22 µm.

In the method of the present disclosure, the size of the pore size in the filtration step may be about 1,000 µm, about 500 µm, about 400 µm, about 300 µm, about 200 µm, about 150 µm, about 100 µm, about 50 µm, about 40 µm, about 30 µm, about 20 µm, about 15 µm, about 10 µm, about 9 µm, about 8 µm, about 7 µm, about 6 µm, about 5 µm, about 4 µm, about 3 µm, about 2 µm, about 1 µm, 0.9 µm, about 0.8 µm, about 0.7 µm, about 0.6 µm, about 0.5 µm, about 0.45 µm, about 0.4 µm, about 0.35 µm, about 0.3 µm, about 0.25 µm, about 0.22 µm, about 0.20 µm, about 0.15 µm, about 0.10 µm, about 0.05 µm, about 0.04 µm, about 0.03 µm, about 0.02 µm, and about 0.01 µm; the range of the pore size in the filtration step may be a range consisted of any two of the above pore sizes, for example but not limited to about 0.05-2 µm, 0.05-1 µm, 0.05-0.8 µm, 0.05-0.6 µm, 0.05-0.4 µm, 0.1-2 µm, 0.1-1 µm, 0.1-0.8 µm, 0.1-0.6 µm, 0.1-0.4 µm, 0.1-0.3 µm, 0.1-0.22 µm, 0.2-1 µm, 0.2-0.8 µm, 0.2-0.6 µm, 0.2-0.4 µm, 0.3-0.8 µm, 0.3-0.6 µm, 0.4-1.0 µm, 0.4-0.8 µm, 0.45-0.8 µm, 0.5-100 µm, 1-100 µm, 1-50 µm, 1-40 µm, 2-50 µm, 2-40 µm, 2-30 µm, 2-20 µm, 2-10 µm, 2-5 µm, 2-4 µm, 3-50 µm, 3-40 µm, 3-30 µm, 3-20 µm, 3-10 µm, 3-5 µm, 3-4 µm, 4-50 µm, 4-40 µm, 4-30 µm, 4-20 µm, 4-10 µm, 4-5 µm, 5-50 µm, 5-40 µm, 5-30 µm, 5-20 µm, 5-10 µm, 6-50 µm, 6-40 µm, 6-30 µm, 6-20 µm, 6-10 µm, 7-50 µm, 7-40 µm, 7-30 µm, 7-20 µm, 7-10 µm, 8-50 µm, 8-40 µm, 8-30 µm, 8-20 µm, 8-10 µm, 9-50 µm, 9-40 µm, 9-30 µm, 9-20 µm, 9-10 µm; preferably the pore sizes of layer-by-layer filtration may be respectively selected from about 1-50 µm, 2-30 µm, 2-20 µm, 6-30 µm, 0.05-1 µm, 0.1-0.8 µm, 0.1-0.22 µm, 0.1-0.3 µm, 0.2-0.8 µm, 0.2-0.6 µm, 0.2-0.4 µm, 0.3-0.8 µm, 0.4-1.0 µm, 0.4-0.8 µm, 0.45-0.8 µm. In some embodiments, more preferably, the pore size in the filtration step may be selected from about 1-50 µm, 0.4-0.8 µm and 0.1-0.3 µm, or a combination thereof, for example about 2-30 µm, about 0.45-0.8 µm, or about 0.1-0.22 µm, for example about 6-30 µm, about 0.45 µm, or about 0.22 µm.

In the method of the present disclosure, the filtration steps in layer-by-layer filtration may be performed in one device, wherein the device includes a plurality of separate units having a filtration function. In some embodiments, layer-by-layer filtration may be performed in two or more devices, wherein each device includes one or more separate units having a filtration function. In some embodiments, layer-by-layer filtration may be performed in one device, wherein the device includes at least 2, 3, 4, 5, 6, 7, 8, 9, 10, 15, 20, 30, 40, 50, 60, 70, 80, 90, 100, 200, 300, 400, and 500 separate units having a filtration function.

In the method of the present disclosure, layer-by-layer filtration includes using a solvent, such as organic solvents and inorganic solvents for conventional extraction, for example water, an isotonic aqueous sodium chloride solution, ethanol, acetone, diethyl ether, petroleum ether, ethyl acetate, or mixtures thereof. In some embodiments, the solvent in layer-by-layer filtration may have a suitable flow rate. For example, the solvent may have a range of about, for example, 1 mL/min, 2 mL/min, 3 mL/min, 4 mL/min, 5 mL/min, 6 mL/min, 7 mL/min, 8 mL/min, 9 mL/min, 10 mL/min, 15 mL/min, 20 mL/min, 25 mL/min, 30 mL/min, 35 mL/min, 40 mL/min, 45 mL/min, 50 mL/min, 60 mL/min, 70 mL/min, 80 mL/min, 90 mL/min, 100 mL/min, 200 mL/min, 300 mL/min, 400 mL/min, 500 mL/min, 600 mL/min, 700 mL/min, 800 mL/min, 900 mL/min, or 1,000 mL/min, or any sub-range within about 1 mL/min to about 1,000 mL/min, for example about 1 mL/min to about 800 mL/min, about 1 mL/min to about 500 mL/min, about 1 mL/min to about 200 mL/min, about 10 mL/min to about 1,000 mL/min, about 10 mL/min to about 800 mL/min, about 10 mL/min to about 500 mL/min, about 10 mL/min to about 200 mL/min, about 20 mL/min to about 1,000 mL/min, about 20 mL/min to about 800 mL/min, about 20 mL/min to about 500 mL/min, about 20 mL/min to about 200 mL/min, about 50 mL/min to about 1,000 mL/min, about 50 mL/min to about 500 mL/min, or about 50 mL/min to about 200 mL/min. In embodiments of large-scale production, the flow rate of the solvent may be as high as about 1 L/min or even higher. In some embodiments, the flow rate of the solvent in the layer-by-layer filtration of the method of the present disclosure is about 20 mL/min to about 1,000 mL/min, for example about 20 mL/min to about 500 mL/min, about 20 to about 200 mL/min, and about 30 to about 100 mL/min.

The layer-by-layer filtration of the present disclosure may be performed at any suitable temperature. For example, the layer-by-layer filtration of the present disclosure may be performed at a suitable temperature, for example about 0°C, 1°C, 2°C, 3°C, 4°C, 5°C, 6°C, 7°C, 8°C, 9°C, 10°C, 11°C, 12°C, 13°C, 14°C, 15°C, 16°C, 17°C, 18°C, 19°C, 20°C, 25°C, 30°C, 35°C, or 40°C, or any sub-range within about 0°C to about 40°C (for example, any range between any two of the above temperatures), for example about 0°C to about 35°C, about 0°C to about 30°C, and about 0°C to about 25°C. In some embodiments, the layer-by-layer filtration of the present disclosure may be performed at ambient temperature (for example 25°C). In other embodiments, the layer-by-layer filtration of the present disclosure may be performed at about 4°C; for example, deep filtration of the layer-by-layer filtration of the present disclosure is performed at about 4°C, and membrane filtration of the layer-by-layer filtration of the present disclosure is performed at about 4°C.

Generally, any suitable filtration material may be used in the layer-by-layer filtration of the present disclosure. For example, the material may be a polymer. In some embodiments, the polymer may be a hydrophobic polymer. In some embodiments, the polymer may be a hydrophilic polymer. In some specific embodiments, the polymer includes, but is not limited to, poly(lactic-co-glycolic acid) (PLGA), polylactic acid (PLA), polyglycolic acid (PGA), polycaprolactone (PCL), polylysine or polyglutamic acid, polyethersulfone (PES) (unmodified), polyethersulfone (mPES), polyvinylidene fluoride (PVDF), cellulose acetate, nitrocellulose, MCE (mixed cellulose ester), ultra-high molecular weight polyethylene (UPE), polytetrafluoroethylene (PTFE), nylon, polysulfone, polyacrylonitrile, polypropylene, polyvinyl chloride, polycarbonate, ceramics, diatomaceous earth, glass fiber, resin-bonded glass fiber, and combinations thereof. Available forms of the filtration material include, but are not limited to, microporous membranes, homogeneous membranes, and asymmetric membranes.

In some embodiments, layer-by-layer filtration includes filtration using a filter membrane, wherein the filter membrane comprises the following polymers: nylon, copolymers of acrylic acid, polysulfone, polyvinylidene fluoride, fiber esters, and cellulose esters. The filter membrane is usually made of polymeric support materials, for example PTFE (polytetrafluoroethylene), PES (polyethersulfone), PVP (polyvinylpyrrolidone), PVDF (polyvinylidene fluoride), nylon (polyamide), PP (polypropylene), cellulose (including cellulose esters), PEEK (polyether ether ketone), nitrocellulose, glass fiber, resin-bonded glass fiber, and the like. In some embodiments, the polymer may be a hydrophilic polymer. Hydrophilic membranes include, but are not limited to, Bioassure (from Cuno); EverLUX ^{™} polyethersulfone; STyLUX ^{™} polyethersulfone (both from Meissner); Millex GV, Millex HP, Millipak 60, Millipak 200, and Durapore CVGL01TP3 membranes (from Millipore); Fluorodyne ^{™} EX EDF membranes, Supor ^{™} EAV, Supor ^{™} EBV, Supor ^{™} EKV (all from Pall); Sartopore ^{™} (from Sartorius); hydrophilic PES membranes from Sterlitech; and WFPES PES membranes from Wolftechnik.

In other embodiments, adsorbents, for example silica particles, diatomaceous earth, or carbon particles, may also be introduced into the layer-by-layer filtration of the present disclosure, for example deep filtration.

In some embodiments, the inherent properties of a membrane may be changed by treating the membrane surface. For example, it is known to prepare hydrophilized or hydrophobized membranes by treating with other materials (for example other polymers, graphite, silicon, and the like) to coat the membrane surface. For example, the membrane used in layer-by-layer filtration may be modified with positive charges; for example, the filtration membrane in layer-by-layer filtration may be a charge-modified polyvinylidene fluoride (PVDF) membrane produced by Micropore, or a membrane obtained from Pall using nylon 66 or positively charged polyethersulfone sulfate. The filter membrane may be sterilized (for example autoclaved) before use to ensure sterility thereof.

### Deep filtration

The present disclosure provides a method for preparing plant exosomes (preferably Dendrobium, more preferably Dendrobium officinale exosomes), which comprises a step of layer-by-layer filtration, wherein the step of layer-by-layer filtration has a function or effect of preventing or avoiding clogging. The method of the present disclosure may use any filtration having a function or effect of preventing or avoiding clogging, for example deep filtration (DF), microfiltration (MF), ultrafiltration (UF), sterile filtration, membrane chromatography (MC), and centrifugation.

In some embodiments, the present disclosure provides a method for preparing plant exosomes (preferably Dendrobium, more preferably Dendrobium officinale exosomes), which contains a step of layer-by-layer filtration, wherein the step of layer-by-layer filtration includes deep filtration. Preferably, in some embodiments, the deep filtration is implemented by a depth filter, for example a depth capsule filter.

In some embodiments, the depth filter is a commercially available depth filter, for example a depth filter produced by Pall Corporation (NY), such as a Supracap ^{™} depth filter; it may also be an activated carbon filter of the MilliStak ^{™} series produced by Millipore (Billerica, MA); or it may also be other depth filters: Profile Star 5 µm depth filter (PALL, Catalog No. BYA050P6), Profile Star 3 µm depth filter (PALL, Catalog No. BYA030P6). In some embodiments, the depth filter used in the present disclosure may be a filtration product from Pall Corporation (NY), such as Supracap^{™} Depth Filter Capsules, and for another example Supracap^{™} 50 depth filter capsules, or Supracap^{™} 100 Depth Filter Capsules.

The load range that may be used for deep filtration in the method of the present disclosure is about 50-200 L/m², for example 50-150 L/m² and 40-100 L/m².

For the present disclosure, the depth filter may be any filter having a deep filtration function. In some embodiments, the depth filter may be a filter having any pore size. In some embodiments, the depth filter used in the present disclosure includes a depth filter having about the following pore size ranges: 0.5-100 µm, 1-100 µm, 1-50 µm, 1-40 µm, 2-50 µm, 2-40 µm, 2-30 µm, 2-20 µm, 3-50 µm, 3-40 µm, 3-30 µm, 3-20 µm, 4-50 µm, 4-40 µm, 4-30 µm, 4-20 µm, 5-50 µm, 5-40 µm, 5-30 µm, 5-20 µm, 6-50 µm, 6-40 µm, 6-30 µm, 7-50 µm, 7-40 µm, 7-30 µm, 7-20 µm, 8-50 µm, 8-40 µm, 8-30 µm, 8-20 µm, 9-50 µm, 9-40 µm, 9-30 µm, and 9-20 µm; preferably, pore sizes of deep filtration may be respectively selected from about 1-50 µm, about 2-30 µm, about 2-20 µm, and about 6-30 µm; more preferably, the depth filter has a pore size of about 2-30 µm, for example a depth capsule filter having a pore size of about 2-30 µm, for example a Supracap^{™} Depth Filter Capsules filter.

For the present disclosure, the depth filter may be any form of depth filter, for example a plate filter, disc filter, cartridge filter, filter stack, depth capsule filter, or cartridge-type filter. In some embodiments, the deep filtration of the present disclosure is performed by a depth capsule filter. Depth capsule filters are generally used in clarification filtration of fermentation broths and cell culture fluids, filtration of serum and blood products, filtration of enzyme preparations, removal of impurities from chemical drugs, decarbonization filtration, or filtration of colloidal or viscous materials. The inventors of the present disclosure have unexpectedly found that depth capsule filters are also suitable for extraction of plant materials, and are particularly suitable for the method of the present disclosure for extracting plant exosomes.

A depth capsule filter may generally comprise a single-layer membrane structure or a double-layer membrane structure. The depth capsule filter used in the method of the present disclosure may be a single-layer membrane structure, which for example comprises cellulose fibers, and/or a filter aid (such as diatomaceous earth and perlite), and/or a resin. In some embodiments, the depth capsule filter comprises two layers of membranes, wherein an upper filter plate has a large pore size, intercepts large-sized particles, and protects a lower filter plate; the lower filter plate has a small pore size, further intercepts small particles, and ensures the clarity of the filtered feed liquid; for example, the pore size of the first-layer membrane may be selected as about 11-30 µm and the pore size of the second-layer membrane may be selected as about 6-15 µm; or the pore size of the first-layer membrane may be about 8-20 µm and the pore size of the second-layer membrane may be about 2-4 µm.

In some embodiments, the material of the depth capsule filter described herein includes any hydrophilic material with low protein adsorption, for example but not limited to polyethersulfone (PES), polyvinylidene fluoride (PVDF), and polypropylene (PP).

For the present disclosure, the deep filtration may be performed using any suitable flow rate. For example, the range of the filtration flow rate that may be used for deep filtration in the method of the present disclosure may be 100-1,000 LMH (the volume of liquid passing through a 1 m² membrane pack per hour), for example about 100 LMH, about 200 LMH, about 500 LMH, about 800 LMH, about 120-500 LMH, about 500-1,000 LMH, or a range composed of any two points between 100-1,000 LMH. Alternatively, the flow rate of the deep filtration of the method of the present disclosure may be expressed in mL/min, for example the deep filtration is performed using a flow rate in a range of about 1 mL/min to about 1,000 mL/min, for example but not limited to about 1 mL/min to about 800 mL/min, about 1 mL/min to about 500 mL/min, about 1 mL/min to about 200 mL/min, about 10 mL/min to about 1,000 mL/min, about 10 mL/min to about 800 mL/min, about 10 mL/min to about 500 mL/min, about 10 mL/min to about 200 mL/min, about 20 mL/min to about 1,000 mL/min, about 20 mL/min to about 800 mL/min, about 20 mL/min to about 500 mL/min, about 20 mL/min to about 200 mL/min, about 50 mL/min to about 1,000 mL/min, about 50 mL/min to about 500 mL/min, or about 50 mL/min to about 200 mL/min . Preferably, the feed rate of the present disclosure is about 20 mL/min to about 1,000 mL/min, for example about 20 mL/min to about 500 mL/min, about 20-200 mL/min.

For the present disclosure, the deep filtration may be performed using any suitable pressure. For example, the deep filtration may be performed using a pressure in a range of about 0.1 psi to about 100 psi, for example about 0.1 psi, 0.5 psi, 1 psi, 2 psi, 3 psi, 4 psi, 5 psi, 6 psi, 7 psi, 8 psi, 9 psi, 10 psi, 20 psi, 30 psi, 40 psi, 50 psi, 60 psi, 70 psi, 80 psi, 90 psi, 100 psi, or any sub-range within about 0.1 psi to about 100 psi (for example, any range between any two of the above pressures).

For the present disclosure, the layer-by-layer filtration (for example deep filtration and optional other filtration steps) may be performed for any suitable period of time. For example, for one production batch, the layer-by-layer filtration may be performed within a time range of about 10 minutes to about 10 hours, for example about 10 minutes, 20 minutes, 30 minutes, 40 minutes, 50 minutes, 60 minutes, 2 hours, 3 hours, 4 hours, 5 hours, 6 hours, 7 hours, 8 hours, 9 hours, 10 hours, or any sub-range within about 10 minutes to about 10 hours (for example, any range between any two of the above time points). Preferably, for example, for 500 ml of plant crude extract obtained by pretreatment, when layer-by-layer filtration including deep filtration and membrane filtration (for example 0.45 µm membrane filtration and 0.22 µm membrane filtration) as defined in the present disclosure is used, the processing time is about 0.5 hour to about 2 hours, preferably about 1 hour, whereas ultracentrifugation of the same plant crude extract by centrifugation at 12,000 g for 70 min twice takes about 3 hours.

The method of the present disclosure may be used to process said plant (preferably Dendrobium, more preferably Dendrobium officinale) at a suitable rate. For example, for one production batch, the rate at which the method of the present disclosure processes plants such as a plant crude extract ranges from about 10 g/hour, 30 g/hour, 50 g/hour, 60 g/hour, 70 g/hour, 80 g/hour, 90 g/hour, 100 g/hour, 200 g/hour, 300 g/hour, 400 g/hour, 500 g/hour, 600 g/hour, 700 g/hour, 800 g/hour, 900 g/hour, 1,000 g/hour, or any sub-range within about 10 g/hour to about 1,000 g/hour (for example, any range between any two of the above production rates).

The method of preparing plant exosomes by layer-by-layer filtration (for example deep filtration and optional other filtration steps) of the present disclosure can obtain about the following numbers of plant exosomes per 100 g of plant: 1×10¹⁰, 5×10¹⁰, 10×10¹⁰, 11×10¹⁰, 12×10¹⁰, 13×10¹⁰, 14×10¹⁰, 15×10¹⁰, 16×10¹⁰, 17×10¹⁰, 18×10¹⁰, 19×10¹⁰, 20×10¹⁰, 22×10¹⁰, 24×10¹⁰, 26×10¹⁰, 28×10¹⁰, 30×10¹⁰, 32×10¹⁰, 34×10¹⁰, 36×10¹⁰, 38×10¹⁰, 40×10¹⁰, 42×10¹⁰, 44×10¹⁰, 46×10¹⁰, 48×10¹⁰, 50×10¹⁰, 52×10¹⁰, 54×10¹⁰, 56×10¹⁰, 58×10¹⁰, 60×10¹⁰, 66×10¹⁰, or even more. In some embodiments, the layer-by-layer filtration of the present disclosure can obtain about 10×10¹⁰-30×10¹⁰, preferably about 10×10¹⁰-20×10¹⁰, for example about 12×10¹⁰ plant exosomes per 100 g of Dendrobium, more preferably Dendrobium officinale.

The depth filter described in the present disclosure comprises deep filtration medium. In some embodiments, the deep filtration medium may be a hydrophilic material with low protein adsorption, such as polyethersulfone (PES), polyvinylidene fluoride (PVDF), polypropylene (PP), and the like (preferably having a pore size range of 1-50 µm). In some embodiments, the deep filtration medium may be in the form of a filter plate. In some embodiments, the deep filtration medium layer is optionally selected from one or more of Supradur P filter plates, K300 filter plates, and BECOPAD P 270 filter plates. Generally, filter plates commonly used in the art are generally divided into two types: one is a filter plate made of synthetic fiber and plant fiber materials; and the other is a filter plate made of asbestos and pulp. Specifically, the filter plate used in the present disclosure may be produced by companies such as 3M, PALL, and EATON in the United States, and commonly used models include the SP series filter plates of 3M, the P series filter plates of PALL, and the BECO series filter plates of EATON.

In some embodiments, the deep filtration medium may be in a gradient distribution or a mixed distribution. In some embodiments, the deep filtration medium may be in a gradient distribution. In some embodiments, the depth capsule filtration device is a double-layer membrane structure in a gradient distribution, wherein the pore sizes of the two-layer membrane structure become smaller sequentially along the flow direction of the filtrate; for example, the pore size of the first-layer membrane may be selected as about 11-30 µm and the pore size of the second-layer membrane may be selected as about 6-15 µm; or the pore size of the first-layer membrane may be about 8-20 µm and the pore size of the second-layer membrane may be about 2-4 µm.

In some embodiments, the deep filtration medium may further comprise a membrane filtration layer, for example the filtration pore size of the membrane filtration layer is preferably about 0.1-10 µm. In some embodiments, the deep filtration medium of the present disclosure may be used in combination with a membrane filtration medium. In some embodiments, the membrane filtration layer is disposed at a liquid outlet end of the deep filtration medium. In some embodiments, the membrane filtration layer is disposed at a liquid inlet end of the deep filtration medium. In some embodiments, the membrane filtration material after depth capsule filtration is generally selected from hydrophilic materials with low protein adsorption, such as polyethersulfone (PES), polyvinylidene fluoride (PVDF), polypropylene (PP), and the like.

In some embodiments, the said deep filtration medium may further comprise a filter aid. In some embodiments, the filter aid may comprise diatomaceous earth, perlite, talc, silica gel, activated carbon, asbestos, molecular sieves, clay, and the like. In some embodiments, the filter aid may be a filter aid based on mineral sources such as silicon dioxide, such as perlite, diatomaceous earth, or sand, or optionally an activated carbon filter aid derived from natural materials such as wood or coconut shell.

In some embodiments, the deep filtration described in the present disclosure may use a pump. In some embodiments, the pump may include a peristaltic pump, diaphragm pump, gear pump, and centrifugal drive pump peristaltic pump. In some embodiments, deep filtration may use a peristaltic pump.

In some embodiments, the flow rate of the pump used for deep filtration may be about the following ranges: 1-1,000 mL/min, 10-1,000 mL/min, 10-800 mL/min, 10-500 mL/min, 15-700 mL/min, 16-600 mL/min, 17-500 mL/min, 18-300 mL/min, 19-400 mL/min, 20-1,000 mL/min, 20-500 mL/min, 20-300 mL/min, 20-200 mL/min, 30-100 mL/min, 40-100 mL/min, or 50-100 mL/min. Preferably, the pump used for deep filtration in the present disclosure is a peristaltic pump, and the flow rate is, for example, about 20-1,000 mL/min, for example about 20-500 mL/min, about 20-200 mL/min, or about 30-100 mL/min.

In some embodiments, an aqueous solution is used in the deep filtration described in the present disclosure to rinse the depth filter; preferably, an isotonic solution is used, more preferably an isotonic sodium chloride solution is used, and most preferably sodium chloride injection is used to rinse the depth capsule filter. For example, in the deep filtration described in the present disclosure, sodium chloride injection is preferably used before filtration starts to rinse the depth capsule filter at a flow rate of, for example, about 20-1,000 mL/min, for example about 20 -500 mL/min, about 20-200 mL/min, or about 30-100 mL/min.

In some embodiments, in deep filtration, an aqueous solution is used to wash the depth filter; preferably, an isotonic solution is used, more preferably an isotonic sodium chloride solution is used, and most preferably sodium chloride injection is used to wash the depth capsule filter. For example, in the deep filtration described in the present disclosure, sodium chloride injection is preferably used at the end of deep filtration to wash the depth capsule filter at a flow rate of, for example, about 20-1,000 mL/min, for example about 20 -500 mL/min, about 20-200 mL/min, or about 30-100 mL/min.

### Membrane filtration

In some embodiments, layer-by-layer filtration further comprises membrane filtration. For example, the layer-by-layer filtration of the present disclosure comprises deep filtration and membrane filtration. For example, in said layer-by-layer filtration, membrane filtration may comprise anti-clogging membrane filtration and sterilizing membrane filtration. In some embodiments, anti-clogging membrane filtration may be performed first, and then sterilizing membrane filtration may optionally be performed. In some embodiments, anti-clogging membrane filtration may be performed before sterilizing membrane filtration. In some embodiments, one or more anti-clogging membrane filtrations may be performed, and then one or more sterilizing membrane filtrations may be performed.

The pore size of membrane filtration of the present disclosure may be about 2 µm, about 1 µm, 0.9 µm, about 0.8 µm, about 0.7 µm, about 0.6 µm, about 0.5 µm, about 0.45 µm, about 0.4 µm, about 0.35 µm, about 0.3 µm, about 0.25 µm, about 0.22 µm, about 0.20 µm, about 0.15 µm, about 0.10 µm, about 0.05 µm, about 0.04 µm, about 0.03 µm, about 0.02 µm, and about 0.01 µm; the pore size range may be a range composed of any two of the above pore sizes, for example but not limited to about the following ranges: 0.05-2 µm, 0.05-1 µm, 0.05-0.8 µm, 0.05-0.6 µm, 0.05-0.4 µm, 0.1-2 µm, 0.1-1 µm, 0.1-0.8 µm, 0.1-0.6 µm, 0.1-0.4 µm, 0.1-0.3 µm, 0.1-0.22 µm, 0.2-1 µm, 0.2-0.8 µm, 0.2-0.6 µm, 0.2-0.4 µm, 0.3-0.8 µm, 0.3-0.6 µm, 0.4-1.0 µm, 0.4-0.8 µm, 0.45-0.8 µm. In some preferred embodiments, the pore size range of membrane filtration may be selected from about 0.4-0.8 µm and about 0.1-0.3 µm or a combination thereof, for example about 0.45-0.8 µm and about 0.1-0.22 µm.

In some embodiments, the layer-by-layer filtration of the present disclosure comprises membrane filtration after deep filtration. For example, membrane filtration may comprise membrane filtration of about 0.4-0.8 µm (for example about 0.45 µm) and membrane filtration of about 0.1-0.3 µm (for example about 0.22 µm). In some embodiments, membrane filtration of about 0.4-0.8 µm (for example about 0.45 µm) may be performed first, and then membrane filtration of about 0.1-0.3 µm (for example about 0.22 µm) may optionally be performed. In some embodiments, membrane filtration of about 0.4-0.8 µm (for example about 0.45 µm) may be performed before membrane filtration of about 0.1-0.3 µm (for example about 0.22 µm). In some embodiments, one or more membrane filtrations of about 0.4-0.8 µm (for example about 0.45 µm) may be performed, and then one or more membrane filtrations of about 0.1-0.3 µm (for example about 0.22 µm) may be performed. Preferably, one or more membrane filtrations, preferably one membrane filtration of about 0.4-0.8 µm (for example about 0.45 µm), may be performed before membrane filtration of about 0.1-0.3 µm (for example about 0.22 µm), so as to reduce or prevent clogging of 0.22 µm membrane filtration.

In some embodiments, the membrane filtration comprises passing through one or more membrane filters connected in series. In some embodiments, the number of membrane filters connected in series ranges from 1 to 10, such as 1, 2, 3, 4, 5, 6, 7, 8, 9 or 10 filters connected in series, preferably 1-4, and more preferably 2-3. The cutoff sizes of all membrane filters connected in series may each be the same or different. In some embodiments, at least 2 of the membrane filters connected in series have different cutoff sizes. In some embodiments, size exclusion decreases from large to small along the membrane filters connected in series. For example, among three membrane filters connected in series, the first filter may be about a 0.45 µm filter, the second filter may be a 0.3 µm filter, and the last filter may be about a 0.22 µm filter.

Exemplary membrane filters used in the method of the present disclosure include, but are not limited to, polyethersulfone membrane filters, polyvinylidene fluoride membrane filters, cellulose membrane filters, mixed cellulose ester membrane filters, cellulose acetate membrane filters, cellulose nitrate membrane filters, polyamide membrane filters, polycarbonate membrane filters, polytetrafluoroethylene membrane filters, polypropylene membrane filters, nitrocellulose membrane filters, glass fiber, resin-bonded glass fiber, or bead filters.

For the method of the present disclosure, the material of the membrane used for membrane filtration may be any filtration material conventionally used in the art, for example the various filtration materials and membrane materials described above in the layer-by-layer filtration section. In some embodiments, the anti-clogging membrane filtration is a resin-bonded glass fiber membrane, preferably having a pore size of about 0.45-0.8 µm. In some embodiments, sterilizing membrane filtration is a polyethersulfone (PES) membrane, preferably having a pore size of about 0.1-0.22 µm.

In some embodiments, the membrane filtration may be performed using any suitable pressure. For example, the membrane filtration may be performed using a feed pressure in a range of about 0.1 psi to about 100 psi, for example about 0.1 psi, 0.5 psi, 1 psi, 2 psi, 3 psi, 4 psi, 5 psi, 6 psi, 7 psi, 8 psi, 9 psi, 10 psi, 20 psi, 30 psi, 40 psi, 50 psi, 60 psi, 70 psi, 80 psi, 90 psi, 100 psi, or any sub-range within about 0.1 psi to about 100 psi (for example, any range between any two of the above feed pressures). The membrane filtration may be performed using any suitable permeate-side pressure. For example, the membrane filtration may be performed using a permeate pressure in a range of about 0.1 psi to about 100 psi, for example about 0.1 psi, 0.5 psi, 1 psi, 2 psi, 3 psi, 4 psi, 5 psi, 6 psi, 7 psi, 8 psi, 9 psi, 10 psi, 20 psi, 30 psi, 40 psi, 50 psi, 60 psi, 70 psi, 80 psi, 90 psi, 100 psi, or any sub-range within about 0.1 psi to about 100 psi (for example, any range between any two of the above pressures).

In some embodiments, the membrane filtration described in the present disclosure may use a pump. In some embodiments, the pump may include a peristaltic pump, diaphragm pump, gear pump, and centrifugal drive pump peristaltic pump. In some embodiments, membrane filtration may use a peristaltic pump. In some embodiments, the flow rate of the pump used for membrane filtration may be in the following ranges: 1-1,000 mL/min, 100-1,000 mL/min, 200-1,000 mL/min, 500-1,000 mL/min, 10-800 mL/min, 10-500 mL/min, 20-1,000 mL/min, 20-500 mL/min, 20-300 mL/min, 20-200 mL/min, 30-100 mL/min, 40-100 mL/min, or 50-100 mL/min. Preferably, the flow rate of the pump for membrane filtration in the present disclosure is about 20-1,000 mL/min, for example about 500-1,000 mL/min, about 20 -500 mL/min, about 20-200 mL/min, or about 30-100 mL/min.

In some embodiments, an aqueous solution, preferably an aqueous isotonic solution, more preferably an isotonic sodium chloride solution, is used in the membrane filtration described in the present disclosure to rinse and/or wash the membrane filter. Sodium chloride injection is preferably used to wash the membrane filter at a flow rate of about 20-1,000 mL/min, for example about 20 --500 mL/min, about 20-200 mL/min, or about 30-100 mL/min.

In some embodiments, the membrane filtration may be performed using any suitable flow rate. For example, the membrane filtration may be performed using a flow rate in a range of about 1 mL/min to about 1,000 mL/min, for example, about 1 mL/min to about 800 mL/min, about 1 mL/min to about 500 mL/min, about 1 mL/min to about 200 mL/min, about 10 mL/min to about 1,000 mL/min, about 10 mL/min to about 800 mL/min, about 10 mL/min to about 500 mL/min, about 10 mL/min to about 200 mL/min, about 20 mL/min to about 1,000 mL/min, about 20 mL/min to about 800 mL/min, about 20 mL/min to about 500 mL/min, about 20 mL/min to about 200 mL/min, about 50 mL/min to about 1,000 mL/min, about 50 mL/min to about 500 mL/min, or about 50 mL/min to about 200 mL/min . In embodiments of large-scale production, the flow rate of membrane filtration may be as high as about 1 L/min or even higher. Preferably, the filtration flow rate of the present disclosure is about 20-1,000 mL/min, for example about 20-200 mL/min or about 30-100 mL/min.

In some embodiments, the membrane filtration may be performed for any suitable time limit. For example, for one production batch, the membrane filtration may be performed within a time range of about 10 minutes to about 10 hours, for example about 10 minutes, 20 minutes, 30 minutes, 40 minutes, 50 minutes, 60 minutes, 2 hours, 3 hours, 4 hours, 5 hours, 6 hours, 7 hours, 8 hours, 9 hours, 10 hours, or any sub-range within about 10 minutes to about 10 hours (for example, any range between any two of the above time points). Preferably, for 500 mL of plant crude extract obtained by pretreatment, membrane filtration is performed for about 1 hour; preferably, when layer-by-layer filtration comprising deep filtration, anti-clogging membrane filtration such as about 0.45 µm filtration, and sterilizing membrane filtration such as about 0.22 µm filtration is used, the processing time is about 0.5 hour to about 2 hours, preferably about 1 hour.

In some embodiments, the method of the present disclosure comprising deep filtration and membrane filtration can obtain about the following yields of plant exosomes per 100 g of plant: 1×10¹⁰, 5×10¹⁰, 10×10¹⁰, 11×10¹⁰, 12×10¹⁰, 13×10¹⁰, 14×10¹⁰, 15×10¹⁰, 16×10¹⁰, 17×10¹⁰, 18×10¹⁰, 19×10¹⁰, 20×10¹⁰, 22×10¹⁰, 24×10¹⁰, 26×10¹⁰, 28×10¹⁰, 30×10¹⁰, 32×10¹⁰, 34×10¹⁰, 36×10¹⁰, 38×10¹⁰, 40×10¹⁰, 42×10¹⁰, 44×10¹⁰, 46×10¹⁰, 48×10¹⁰, 50×10¹⁰, 52×10¹⁰, 54×10¹⁰, 56×10¹⁰, 58×10¹⁰, 66×10¹⁰, or even more. In some embodiments, the membrane filtration of the present disclosure can obtain about 10×10¹⁰-20×10¹⁰, preferably about 12×10¹⁰, plant exosomes per 100 g of Dendrobium (preferably Dendrobium officinale).

### Preferred embodiments

In one embodiment, the method of the present disclosure for preparing plant exosomes comprises the following steps:
(a) obtaining a plant raw material;
(b) pretreating the obtained plant raw material to obtain a plant crude extract solution;
(c) subjecting the plant crude extract solution to layer-by-layer filtration treatment;
wherein the layer-by-layer filtration treatment comprises a deep filtration step and an optional membrane filtration step.

In one embodiment, the method of the present disclosure for preparing plant exosomes comprises the following steps:
(a) obtaining a plant raw material;
(b) pretreating the obtained plant raw material, preferably by mixing, pulverizing, filtering, and centrifuging, to obtain a plant crude extract solution;
(c) subjecting the plant crude extract solution to layer-by-layer filtration treatment to obtain a filtrate;
   wherein the layer-by-layer filtration treatment comprises a deep filtration step, preferably the deep filtration step uses deep filtration having a pore size of about 1-50 µm, more preferably deep filtration having a pore size of about 2-30 µm; and
(d) subjecting the filtrate in step (c) to membrane filtration, preferably anti-clogging membrane filtration and/or sterilizing membrane filtration, for example anti-clogging membrane filtration having a pore size of about 0.4-0.8 µm and/or sterilizing membrane filtration having a pore size of about 0.1-0.3 µm, more preferably membrane filtration having a pore size of about 0.45-0.8 µm and membrane filtration having a pore size of about 0.1-0.22 µm, most preferably the membrane filtration consists of sequentially performed 0.45 µm anti-clogging membrane filtration and 0.22 µm sterilizing membrane filtration, to obtain plant exosomes.

In the above embodiments, in said step (b), the obtained plant material is pretreated, wherein the pretreatment comprises mixing said plant material with an isotonic solution (preferably sodium chloride injection), pulverizing the plant raw material using a wall-breaking machine to obtain plant residue liquid, filtering said plant residue liquid with a filter screen to obtain plant juice, and centrifuging said plant juice followed by collecting the supernatant to obtain a plant crude extract solution.

In the above embodiments, in said step (b), plant material may be subjected to large-scale mixing and pulverizing. In some specific embodiments, in said step (b), plants having a mass of about 1,000 g or even more, for example about 500-1,000 g, about 50-200 g, for example about 100 g, are subjected to mixing and pulverizing. In some embodiments, said step (b) is performed at about 4°C.

In the above embodiments, in said step (b), the pulverized residue liquid is stirred, preferably at a stirring speed of about 50-500 rpm, more preferably about 100-250 rpm, and still more preferably about 200±20 rpm. In some embodiments, said stirring time is about 10-200 min, preferably about 2-100 min, and more preferably about 30 min.

In some preferred embodiments, the centrifugation in said step (b) is performed at about 1,000-10,000 g. In some preferred embodiments, the centrifugation in said step (b) is performed at least about 3,000-6,000 g. In some preferred embodiments, the centrifugation time in said step (b) is about 5-60 min. In some preferred embodiments, the ultracentrifugation time in said step (b) is about 15 min, and/or is performed at about 4°C.

In one specific embodiment of the above method for preparing plant exosomes, said plant material is or comprises Dendrobium, preferably Dendrobium officinale.

In one specific embodiment of the above method for preparing plant exosomes, said layer-by-layer filtration treatment comprises a deep filtration step, wherein said depth filter is depth capsule filtration having a pore size of about 1-50 µm, preferably about 2-30 µm, and a loading range of about 50-200 L/m², for example 50-150 L/m² or 40-100 L/m²; preferably the depth filter is a depth filter produced by Pall Corporation (NY), namely Supracap^{™} Depth Filter Capsules.

In one specific embodiment of the above method for preparing plant exosomes, in step (d), the filtrate in step (c) is sequentially subjected to about 0.45 µm filtration and about 0.22 µm filtration, and the final filtrate is collected to obtain plant exosomes.

### Technical effects

Compared with the ultracentrifugation method, the layer-by-layer filtration method of the present disclosure has the following advantages:
(1) lower time consumption;
(2) higher exosome yield or particle concentration;
(3) being capable of preventing filter membrane clogging caused by excessive impurities, and being capable of ensuring sterility of the product;
(4) the extracted plant exosomes have excellent metabolomic performance;
(5) the extracted plant exosomes have a typical exosome-like saucer shape, with a particle size in the range of 30-200 nm;
(6) the extracted plant exosomes have anti-inflammatory and immunomodulatory activities.

The method of the present disclosure for preparing plant exosomes (preferably Dendrobium, more preferably Dendrobium officinale exosomes) is suitable for small-scale, pilot-scale, and large-scale production. In some embodiments, the exosome preparation method of the present disclosure is particularly used for large-scale production, and the amount of plant material that can be processed is, for example, at least about 100 g, 200 g, 300 g, 400 g, 500 g, 600 g, 700 g, 800 g, 900 g, 1,000 g, 2,000 g, 3,000 g, 4,000 g, 5,000 g, 6,000 g, 7,000 g, 8,000 g, 9,000 g, 10,000 g, 50,000 g, or even up to 100,000 g. Preferably, the exosome preparation method of the present disclosure can process about 1,000 g or less of plant material, more preferably can process about 10,000 g or less of plant material, and most preferably can process about 100,000 g or less of plant raw material.

In particular, the layer-by-layer filtration treatment of the present disclosure is particularly suitable for large-scale production; for example, large-scale production is achieved by increasing the membrane area of the filter based on the deep filtration and membrane filtration preferred in the present disclosure (as shown in the Examples). By appropriately increasing the membrane area in layer-by-layer filtration, the layer-by-layer filtration of the present disclosure can filter at least about 100 g, 200 g, 300 g, 400 g, 500 g, 600 g, 700 g, 800 g, 900 g, 1,000 g, 2,000 g, 3,000 g, 4,000 g, 5,000 g, 6,000 g, 7000 g, 8,000 g, 9,000 g, 10,000 g, 50,000 g, or even 100,000 g of plant material or a corresponding pretreatment liquid thereof.

### III. Exosomes

The plant exosomes (preferably Dendrobium, more preferably Dendrobium officinale exosomes) prepared according to the method of the present disclosure may have any suitable hydrodynamic particle size or diameter. For example, the plant exosomes may have a hydrodynamic particle size or diameter of about 10 nm to about 10 µm, for example about 10 nm, 20 nm, 30 nm, 40 nm, 50 nm, 60 nm, 70 nm, 80 nm, 90 nm, 100 nm, 110 nm, 120 nm, 130 nm, 140 nm, 150 nm, 200 nm, 300 nm, 400 nm, 500 nm, 600 nm, 700 nm, 800 nm, 900 nm, 1 µm, 2 µm, 3 µm, 4 µm, 5 µm, 6 µm, 7 µm, 8 µm, 9 µm, and 10 µm, or any sub-range within about 10 nm to about 10 µm (for example, any range between any two of the above particle sizes). Preferably, the plant exosomes prepared according to the method of the present disclosure have a diameter of about 30-450 nm, preferably a diameter of about 30-250 nm, and more preferably have a diameter of about 30-200 nm.

Said plant exosomes may have any suitable shape, including but not limited to a saucer shape, spherical shape, or disc shape. Preferably, the plant exosomes prepared according to the method of the present disclosure have a saucer shape.

Compared with plant exosomes prepared by the ultracentrifugation method, the plant exosomes (preferably Dendrobium, more preferably Dendrobium officinale exosomes) prepared according to the method of the present disclosure have more metabolites in one or more of the following aspects: lipids and lipid-like molecules, organic oxygen compounds, phenylpropanoids and polyketides, organicheterocyclic compounds, organic acids and derivatives thereof, and benzenoids. In some embodiments, the number of lipids in the plant exosomes prepared according to the method of the present disclosure is greater than the number of lipids in the plant exosomes prepared by the ultracentrifugation method. As shown in Table 4 of the Examples below, the lipid classifications mainly include fatty acyls (fatty acyl, FA), glycerolipids (GL), glycerophospholipids (GP), prenol lipids (PR), sphingolipids (SP), steroids (ST), and saccharolipids (SL). In some embodiments, the number of species of fatty acyl compounds in the plant exosomes prepared according to the method of the present disclosure is 190-300, preferably 200-250. In some embodiments, the number of species of glycerolipid compounds in the plant exosomes prepared according to the method of the present disclosure is 2-10, preferably 3-9, and more preferably 4-8. In some embodiments, the number of species of glycerophospholipid compounds in the plant exosomes prepared according to the method of the present disclosure is 2-10, preferably 3-8, and more preferably 4-7. In some embodiments, the number of species of prenol lipid compounds in the plant exosomes prepared according to the method of the present disclosure is 190-400, preferably 200-350, and more preferably 200-300. In some embodiments, the number of species of sphingolipid compounds in the plant exosomes prepared according to the method of the present disclosure is 2-9, preferably 2-5, and more preferably 3. In some embodiments, the number of species of steroid compounds in the plant exosomes prepared according to the method of the present disclosure is 10-100, preferably 20-50, and more preferably 20-40. In some embodiments, the number of species of saccharolipid compounds in the plant exosomes prepared according to the method of the present disclosure is 0-5, preferably 2-5, and most preferably 2-3.

In some embodiments, the Dendrobium, preferably Dendrobium officinale exosomes provided by the present disclosure comprise about 1×10⁵ to 1×10¹⁰ particles/mL, preferably 1×10⁶ to 1×10⁹ particles/mL, more preferably 1×10⁷ to 1×10⁹ particles/mL, for example about 1×10⁸ particles/mL.

In some embodiments, the Dendrobium exosomes, preferably Dendrobium officinale exosomes, provided by the present disclosure comprise one or more of the following metabolites: lipids and lipid-like molecules, organic oxygen compounds, phenylpropanoids and polyketides, organicheterocyclic compounds, organic acids and derivatives thereof, and benzenoids. In some embodiments, the Dendrobium exosomes, preferably Dendrobium officinale exosomes, provided by the present disclosure at least comprise lipids and lipid-like molecules and organic oxygen compounds, and lipids and lipid-like molecules together with organic oxygen compounds account for about 40%, 45%, 50%, or 60% or more of the total number of metabolites, for example about 50% of the total number of metabolites; preferably, lipids and lipid-like molecules account for 30%-40% of the total number of metabolites, for example about 35%, and organic oxygen compounds account for 10%-20% of the total number of metabolites, for example about 15%.

In some embodiments, the Dendrobium exosomes, preferably Dendrobium officinale exosomes, provided by the present disclosure comprise lipids and lipid-like molecules, organic oxygen compounds, and phenylpropanoids and polyketides, wherein lipids and lipid-like molecules account for 30%-40% of the total number of metabolites, for example about 35%; organic oxygen compounds account for 10%-20% of the total number of metabolites, for example about 15%; and phenylpropanoids and polyketides account for 10%-20% of the total number of metabolites, for example about 14%.

In some embodiments, the Dendrobium exosomes, preferably Dendrobium officinale exosomes, provided by the present disclosure comprise lipids and lipid-like molecules, organic oxygen compounds, and phenylpropanoids and polyketides, preferably wherein lipids and lipid-like molecules account for 30%-40% of the total number of metabolites, for example about 35%; organic oxygen compounds account for 10%-20% of the total number of metabolites, for example about 15%; and phenylpropanoids and polyketides account for 10%-20% of the total number of metabolites, for example about 14%.

In some embodiments, the Dendrobium exosomes, preferably Dendrobium officinale exosomes, prepared according to the method of the present disclosure comprise lipids and lipid-like molecules, organic oxygen compounds, phenylpropanoids and polyketides, organicheterocyclic compounds, organic acids and derivatives thereof, and benzenoids, preferably wherein lipids and lipid-like molecules account for 30%-40% of the total number of metabolites, for example about 35%; organic oxygen compounds account for 10%-20% of the total number of metabolites, for example about 15%; phenylpropanoids and polyketides account for 10%-20% of the total number of metabolites, for example about 14%; organicheterocyclic compounds account for 7%-17% of the total number of metabolites, for example about 12%; organic acids and derivatives thereof account for 5%-15% of the total number of metabolites, for example about 10%; and benzenoids account for 4%-10% of the total number of metabolites, for example about 8%.

Compared with plant exosomes prepared by the ultracentrifugation method, the plant exosomes (preferably Dendrobium, more preferably Dendrobium officinale exosomes) prepared according to the method of the present disclosure have metabolically up-regulated pathways (FIG. 5), such as tryptophan metabolism, arachidonic acid metabolism, phenylpropanoid biosynthesis, starch and sucrose metabolism, alanine, aspartate and glutamate metabolism, galactose metabolism, ABC transporters, valine, leucine and isoleucine biosynthesis, cutin, suberine and wax biosynthesis, linoleic acid metabolism, β-alanine metabolism, nucleotide metabolism, histidine metabolism, aminoacyl-tRNA biosynthesis, arginine biosynthesis, and biosynthesis of various secondary metabolites.

Specifically, compared with Dendrobium exosomes prepared by the ultracentrifugation method, the Dendrobium exosomes prepared according to the method of the present disclosure have 73 pathways related to differential metabolites, and a total of 20 significantly enriched pathways. Compared with Polygonati Rhizoma exosomes prepared by the ultracentrifugation method, Polygonati Rhizoma exosomes prepared according to the disclosed method have 82 pathways related to differential metabolites, and a total of 27 significantly enriched pathways.

In some embodiments, compared with Dendrobium exosomes prepared by the ultracentrifugation method, the Dendrobium exosomes prepared according to the method of the present disclosure have a total of 770 significantly differential metabolites, including 537 up-regulated metabolites and 233 down-regulated metabolites.

In some embodiments, in an analysis after plant exosomes are stained in vitro using the lipophilic dye PKH67, the positive rate of the Dendrobium exosomes prepared according to the method of the present disclosure is 97.4%. In detection of protein content of plant exosomes, the protein content of the Dendrobium exosomes prepared according to the method of the present disclosure is 875.89 µg/mL.

In some embodiments, the Dendrobium exosomes prepared by the method of the present disclosure show a good anti-inflammatory effect, and the TNF-α inhibition rate shown in the Examples is about 60% or more, even close to 100%.

### IV. Composition

The present disclosure provides a plant exosome composition, comprising Dendrobium (preferably Dendrobium officinale) exosomes, preferably Dendrobium (preferably Dendrobium officinale) exosomes prepared by the method for preparing plant exosomes as generally, preferably or specifically defined in the present disclosure.

Specifically, the plant exosome composition provided by the present disclosure is a pharmaceutical composition, which, in addition to comprising Dendrobium (preferably Dendrobium officinale) exosomes (preferably Dendrobium exosomes prepared by the method for preparing plant exosomes as generally, preferably or specifically defined in the present disclosure), optionally further comprises a pharmaceutically acceptable carrier or other active ingredient. Said carrier is commonly used in pharmaceutical preparations and includes, but is not limited to, lactose, dextrose, sucrose, sorbitol, mannitol, starch, gum arabic, calcium phosphate, alginate, gelatin, calcium silicate, microcrystalline cellulose, polyvinylpyrrolidone, cellulose, water, syrup, methylcellulose, methyl hydroxybenzoate, propyl hydroxybenzoate, talc, magnesium stearate, and mineral oil.

Specifically, the plant exosome composition provided by the present disclosure is a food composition or a nutrition and health-care composition, which comprises Dendrobium (preferably Dendrobium officinale) exosomes, preferably Dendrobium (preferably Dendrobium officinale) exosomes prepared by the method for preparing plant exosomes as generally, preferably or specifically defined in the present disclosure. When the food composition or nutrition and health-care composition of the present disclosure is used as a food additive, said composition may be directly added and used together with other foods or food ingredients according to conventional methods. Generally, when preparing a food or beverage, the food composition or nutrition and health-care composition of the present disclosure may be added in an amount of about 15 parts by weight or less, preferably about 10 parts by weight or less, based on the total weight of the composition.

The nutrition and health-care composition of the present disclosure itself may be in the form of a nutritional supplement or health product, and in addition to the Dendrobium (preferably Dendrobium officinale) exosomes of the present disclosure, may further contain other nutrients and/or nutraceutically acceptable excipients or carriers, such as various proteins, fats, vitamins, prebiotics, probiotics, electrolytes, flavoring agents, coloring agents, pectic acid and salts thereof, alginic acid and salts thereof, organic acids, protective colloid thickeners, pH regulators, stabilizers, preservatives, glycerol, alcohol, carbonates used in carbonated beverages, and the like.

Foods to which the above food composition or nutrition and health-care composition may be added include, but are not limited to, meat, sausage, bread, chocolate, sugars, fast foods, biscuits, pizza, ramen, other noodles, chewing gums, dairy products including ice cream products, various soups, beverages, tea beverages, alcoholic beverages, vitamin complexes, and the like. In some embodiments, the food composition of the present disclosure may be prepared into a functional food.

The plant exosome composition (pharmaceutical composition/nutrition and health-care composition/food composition) of the present disclosure may be in different forms for oral administration, such as capsules (including gelatin capsules, soft capsules, and hard capsules), tablets (including sugar-coated tablets, tablets, pills, powders, granules, and chewing gums), solutions, suspensions, emulsions, syrups, elixirs, polysaccharide films, gels or gelatin, and any other form known to those skilled in the art; it may also be administered by a topical or transdermal route, or by any other suitable route (such as a parenteral route).

Specifically, the exosome composition provided by the present disclosure is a beauty/cosmetic composition, which comprises Dendrobium (preferably Dendrobium officinale) exosomes, preferably Dendrobium (preferably Dendrobium officinale) exosomes prepared by the method for preparing plant exosomes as generally, preferably or specifically defined in the present disclosure. The beauty/cosmetic composition of the present disclosure generally comprises at least one cosmetically acceptable excipient or auxiliary material. Said "cosmetically acceptable excipient or auxiliary material" may be selected from: solvents, solubilizers, preservatives, antioxidants, pH regulators, penetration enhancers, liposomes, humectants, thickeners, chelating agents, skin feel modifiers, surfactants, emulsifiers, propellants, fragrances, pigments, and other functional additives. The form of said beauty composition includes, for example but is not limited to, toilet soap, soap, facial cleanser, cleansing foam, cleansing lotion, cleansing cream, shower gel, skin softening water, skin care gel, skin care lotion, skin care cream, essence, eye cream, facial mask, aerosol or spray, lotion, skin softener, toner, astringent, emulsion, milky lotion, moisturizing lotion, nourishing lotion, massage cream, nourishing cream, moisturizing cream, hand cream, foundation, essence, nourishing essence, pressed powder, body lotion, cleansing lotion, and the like. These forms of the beauty composition may be prepared by methods well known to those skilled in the art.

When the beauty composition of the present disclosure is a paste, cream, or gel, animal fibers, plant fibers, waxes, paraffin, starch, tragacanth, cellulose derivatives, polyethylene glycol, silica gel, bentonite, silicon dioxide, talc, zinc oxide, and the like may be used as carrier components. When the beauty composition of the present disclosure is a powder or spray, lactose, talc, silicon dioxide, aluminum hydroxide, calcium silicate, polyamide powder, and the like may be used as carrier components. In particular, when the preparation is a spray, it may further comprise a propellant such as chlorofluorocarbon, propane/butane, or dimethyl ether. When the beauty composition of the present disclosure is a solution or emulsion, a solvent, solubilizer, or emulsifier may be used as a carrier component. For example, water, ethanol, isopropanol, ethyl carbonate, ethyl acetate, benzyl alcohol, benzyl benzoate, propylene glycol, 1,3-butanediol oil, glycerol aliphatic ester, polyethylene glycol, or sorbitan fatty acid ester may be used. When the beauty composition of the present disclosure is a suspension, liquid diluents such as water, ethanol, or propylene glycol, suspending agents such as ethoxylated isostearyl alcohol, polyoxyethylene sorbitol ester and polyoxyethylene sorbitan ester, microcrystalline cellulose, aluminum metahydroxide, bentonite, agar, tragacanth, and the like may be used as carrier components.

### V. Uses

The exosomes or composition for any use of the present disclosure may include the exosomes or composition that are general or specific in the present disclosure, preferably the exosomes of Part III or the composition of Part IV of the present disclosure. Those skilled in the art will understand that the exosomes for any use of the present disclosure may freely combine all features disclosed herein; for example, the exosomes of Part III of the present disclosure may be exosomes having any combination of features such as different particle densities, different metabolite species, and different percentages of metabolite species as defined.

Many diseases are accompanied by inflammatory responses, including skin-related inflammation (such as dermatitis, acne, pimples, chloasma, and the like), and therefore reducing the level of inflammation will be beneficial for disease treatment and care. Accordingly, in some embodiments, the present disclosure provides use of plant exosomes (preferably Dendrobium, more preferably Dendrobium officinale exosomes) or a composition comprising the same, for treating or preventing, or helping/assisting in improving, alleviating, or controlling inflammation, for example skin inflammation. In other embodiments, the present disclosure provides use of plant exosomes (preferably Dendrobium, more preferably Dendrobium officinale exosomes) or a composition comprising the same, for or helping/assisting in cosmetic, non-therapeutic treatment and/or care of skin, hair, nails and/or mucosa, preferably for or helping/assisting in cosmetic, non-therapeutic treatment and/or care of inflammation of skin, hair, nails and/or mucosa.

In some embodiments, the plant exosomes of the present disclosure (preferably Dendrobium, more preferably Dendrobium officinale exosomes or a composition comprising the same may be used in the skin field for uses such as repair, anti-inflammation, antioxidation, anti-aging, whitening, moisturizing, firming and anti-wrinkle. For example, the plant exosomes or a composition comprising the same may be used for anti-inflammatory use, preferably anti-inflammatory use in the skin field. For another example, the plant exosomes or a composition comprising the same may be used for antioxidation. For another example, the plant exosomes or a composition comprising the same may be used for repair in the skin field. For another example, the plant exosomes or a composition comprising the same may be used for anti-aging. For another example, the plant exosomes or a composition comprising the same may be used for whitening. For another example, plant exosomes or a composition comprising the same may be used for moisturizing.

Thus, the present disclosure also provides use of plant exosomes (preferably Dendrobium, more preferably Dendrobium officinale exosomes) or a composition comprising the same in preparing a product (medicine, food, nutraceutical, beauty/cosmetic product). In some embodiments, the medicine is used for treating or preventing inflammation, for example dermatitis, acne, pimples, chloasma, and the like. In some embodiments, the nutraceutical helps or assists in antioxidation, repair, anti-aging, whitening, firming and anti-wrinkle, and/or moisturizing.

The present disclosure further provides a method for treating or preventing inflammation, preferably skin inflammation, or treating or preventing dermatological symptoms, or assisting in improving, alleviating or controlling dermatological symptoms (specifically for repair, anti-inflammation, antioxidation, anti-aging, whitening, firming and anti-wrinkle, or moisturizing), comprising administering to a subject in need thereof plant exosomes (preferably Dendrobium, more preferably Dendrobium officinale exosomes) prepared by the aforementioned generally or preferably defined method or a composition comprising the same (pharmaceutical composition/food composition/beauty or cosmetic composition/nutrition and health-care composition).

In some embodiments, the plant exosomes provided by the present disclosure (preferably Dendrobium, more preferably Dendrobium officinale exosomes) or a composition comprising the same have an anti-aging effect on skin. In some embodiments, the plant exosomes provided by the present disclosure (preferably Dendrobium, more preferably Dendrobium officinale exosomes) or a composition comprising the same have an anti-aging effect on skin cells, such as fibroblasts. In some embodiments, the plant exosomes provided by the present disclosure (preferably Dendrobium, more preferably Dendrobium officinale exosomes) or a composition comprising the same have an effect of improving or reducing skin aging caused by ultraviolet irradiation or UV-induced cellular senescence. In some embodiments, the plant exosomes provided by the present disclosure (preferably Dendrobium, more preferably Dendrobium officinale exosomes) or a composition comprising the same have an effect, in skin cells (preferably UV-irradiated skin cells), of promoting expression of type I collagen and/or type IV collagen, reducing cyclobutane-pyrimidone dimer (CPD), or increasing hyaluronic acid (HA). For example, the Dendrobium officinale exosomes prepared by the present disclosure have anti-aging or an effect of promoting expression of type I collagen, have an effect of promoting type I collagen (COL I) and/or type IV collagen (COL IV), reducing cyclobutane-pyrimidone dimer (CPD), or increasing hyaluronic acid (HA).

In some embodiments, the plant exosomes provided by the present disclosure (preferably Dendrobium, more preferably Dendrobium officinale exosomes) or a composition comprising the same have a firming and anti-wrinkle effect on skin, preferably said skin is UV-irradiated. In some embodiments, the plant exosomes provided by the present disclosure (preferably Dendrobium, more preferably Dendrobium officinale exosomes) or a composition comprising the same can increase or restore the thickness of the epidermal viable cell layer or increase collagen fibers. In some embodiments, the Dendrobium officinale exosomes provided by the present disclosure or a composition comprising the same can improve thinning of the epidermal viable cell layer and reduction of collagen fibers in skin caused by UV irradiation, and/or maintain skin elasticity. In some embodiments, the plant exosomes provided by the present disclosure (preferably Dendrobium, more preferably Dendrobium officinale exosomes) or a composition comprising the same can up-regulate the contents of laminin (such as LN 5), nidogen, integrin α6β4, and/or reticulin. In some embodiments, the Dendrobium officinale exosomes provided by the present disclosure or a composition comprising the same can up-regulate the contents of LN 5, nidogen, integrin α6β4, and/or reticulin, so as to achieve a firming and anti-wrinkle effect. In some embodiments, the Dendrobium officinale exosomes provided by the present disclosure or a composition comprising the same have a firming and anti-wrinkle effect on UV-irradiated skin.

In some embodiments, the plant exosomes provided by the present disclosure (preferably Dendrobium, more preferably Dendrobium officinale exosomes) or a composition comprising the same can improve or reduce UVA-induced cell enlargement, or reduce the percentage of UVA-induced enlarged cells. In some embodiments, the plant exosomes provided by the present disclosure (preferably Dendrobium, more preferably Dendrobium officinale exosomes) or a composition comprising the same have effects of anti-photoaging, restoring skin elasticity, and removing wrinkles. In some embodiments, the plant exosomes provided by the present disclosure (preferably Dendrobium, more preferably Dendrobium officinale exosomes) or a composition comprising the same have transdermal properties, or penetrate through the stratum corneum epidermal layer to exert their active effects.

In some embodiments, the Dendrobium officinale exosomes provided by the present disclosure or a composition comprising the same act in the preparation of a product/cosmetic/composition/ beauty product/medicine having antioxidation, repair, anti-aging, whitening, firming and anti-wrinkle, and/or moisturizing effects, preferably an anti-aging effect on skin or a firming and anti-wrinkle effect; optionally, said Dendrobium officinale exosomes or a composition comprising the same is the sole active ingredient having antioxidation, repair, anti-aging, whitening, firming and anti-wrinkle, and/or moisturizing effects, preferably an anti-aging effect on skin or a firming and anti-wrinkle effect.

For the above methods and uses of the present disclosure, the plant exosomes or a composition comprising the same may be administered by oral administration, topical administration, or parenteral administration. In particular, topical or transdermal application may be implemented by iontophoresis, sonophoresis, electroporation, mechanical pressure, osmotic pressure gradient, occlusive treatment, microinjection, microneedles (or microneedle arrays), needle-free injection by pressure, microelectric patches, facial masks, or any combination thereof. Some techniques that create channels in the skin (for example, microneedles or lasers) themselves serve as treatment means and can be used in combination with therapy using plant exosomes or a composition comprising the same, thereby helping the plant exosomes or composition comprising the same to be absorbed and reach deeper skin layers and enhancing their activity. The local area of such combination treatment will be determined by the nature of the condition, disorder, and/or disease to be treated and/or handled.

The daily administration dose and administration frequency of said plant exosomes (preferably Dendrobium (more preferably Dendrobium officinale) exosomes) or a composition comprising the same may vary depending on various factors, such as the stage of the disease to be treated, age, health condition, the presence of complications, and the like. In one aspect, said composition may be administered, for example, at a daily dose of 1 µg/kg to 200 mg/kg, more specifically 50 µg/kg to 50 mg/kg, 1-3 times per day. However, the administration dose does not limit the scope of the present disclosure in any way.

The plant exosomes of the present disclosure also have uses in treating various diseases in mammals, such as humans. In some embodiments, said plant exosomes have uses in treating or preventing diseases related to the following systems: respiratory system, digestive system, circulatory system, nervous system, endocrine system, genitourinary system, or musculoskeletal system. In some embodiments, said treating or preventing a disease related to the respiratory system comprises treating COVID-19, inhibiting pulmonary inflammation, treating pulmonary fibrosis, or preventing pulmonary fibrosis and pulmonary inflammation. In some embodiments, said treating or preventing a disease related to the digestive system comprises promoting recovery from colitis, treating colon cancer, treating acute and chronic colitis, treating ulcerative colitis, treating chronic periodontitis, preventing alcohol-induced liver damage, treating IBD and inhibiting CAC progression, preventing colitis, preventing colitis caused by dextran sulfate sodium, treating DSS-induced colitis, inhibiting liver metastasis of colon cancer, inhibiting Clostridioides difficile infection, treating liver inflammation, preventing GalN/LPS-induced acute liver injury, inhibiting proliferation of hepatocellular carcinoma cells, or inhibiting growth of tumor cells. In some embodiments, said treating or preventing a disease related to the circulatory system comprises improving doxorubicin-induced myocardial injury, preventing damage to the vascular system caused by various stressors, or preventing oxidative stress in human mesenchymal stromal cells. In some embodiments, said treating or preventing a disease related to the nervous system comprises treating glioma, treating brain glioma, inhibiting development of glioma, or stimulating neural differentiation of stem cells. In some embodiments, said treating or preventing a disease related to the endocrine system comprises preventing insulin resistance and obesity, or preventing or inhibiting obesity-related gastrointestinal inflammation caused by a high-fat and high-sugar diet. In some embodiments, said treating or preventing a disease related to the genitourinary system comprises inhibiting occurrence and development of mammalian breast cancer, or treating cervical cancer. In some embodiments, said treating or preventing a disease related to the musculoskeletal system comprises promoting wound healing. In addition, the plant exosomes of the present disclosure may also serve as a drug delivery tool. In some embodiments, said plant exosomes may serve as a drug delivery tool to transport various drug molecules for treating the above diseases, for example MTX for treating colitis, PTX for treating colon cancer, and DOX for treating glioblastoma. In some embodiments, said plant exosomes may serve as a delivery tool for treating plant diseases caused by fungal infection. In some embodiments, different plant exosomes have different properties, such as variations in size, charge, and stability. These properties inevitably affect loading of drugs in plant exosomes. For example, in certain embodiments, the plant exosomes serving as a drug delivery tool are plant exosomes having a smaller size, for example 30-80 µm, 30-60 µm, or 30-50 µm. Due to a higher surface-area-to-volume ratio, said plant exosomes having a smaller size have a greater drug loading capacity, a faster release rate, and higher stability. In other embodiments, plant exosomes may serve as a drug delivery tool to transport positively charged drug molecules. Due to the presence of phosphates, plant exosomes generally carry a negative charge, and therefore, under the influence of electrostatic attraction, positively charged molecules are more efficiently absorbed and encapsulated by these negatively charged or neutral plant exosomes.

### Examples

The technical solutions of the present disclosure are further described below in conjunction with specific examples, but the protection scope of the present disclosure is not limited to these examples. Any changes or equivalent substitutions that do not depart from the concept of the present disclosure are included within the protection scope of the present disclosure.

Experimental methods and means for which specific conditions are not indicated in the following examples are generally carried out according to conventional conditions for such experimental operations in the art. Experimental equipment for which specific sources and models are not indicated in the following examples is conventional equipment in the art well known to those skilled in the art, and can be conventionally selected and operated by those skilled in the art . Experimental materials and reagents used in the following examples, unless otherwise specified, are commercially available and can be used directly without further purification. Unless otherwise specified, percentages and parts are weight percentages and parts by weight, respectively. Unless otherwise specified, ratios of liquids are volume ratios, and temperatures used in the present disclosure are in degrees Celsius °C.

### 1. Experimental methods

### 1.1 Preparation of plant crude extract

Dendrobium officinale was thoroughly washed, and the moisture was wiped off with a clean cloth. The plant to be treated was weighed, and according to a mass-volume ratio (m/v, g/mL), plant mass: sodium chloride injection volume = 1:3-1:20, the weighed plant was mixed with sodium chloride injection. The plant was pulverized using a wall-breaking machine, and the pulverized plant residue liquid was collected into a measuring cylinder. The measuring cylinder was placed on an ice medium, and stirring was performed for 30 min using a mixer at a rotation speed of 200±20 rpm. A filter screen was used, into which the plant residue liquid was poured, so that the plant residues were separated from the juice, and the plant juice was collected. The collected plant juice was centrifuged at 2,000-5,000 g for 10-35 min under conditions of 4°C. In a biosafety cabinet, the precipitate was discarded, and the supernatant P1 was collected as the plant crude extract.

### 1.2 Isolation and extraction of plant exosomes using a layer-by-layer filtration method

S1 Deep filtration: In a biosafety cabinet, a peristaltic pump, pump tubing, and an approximately 2-30 µm depth capsule filter (Supracap ^{™} Depth Filter Capsules, Pall, filtration loading capacity of about 50-200 L/m²) were connected together. The depth capsule filter was rinsed with sodium chloride injection at a flow rate of 20-200 mL/min, and finally the liquid was drained. P1 was filtered at a flow rate of 20-200 mL/min, and filtrate P2 was collected. The filter was removed, the tubing was washed with sodium chloride injection at a flow rate of 20-200 mL/min, and finally the liquid was drained and the k pump tubing was detached.

S2 0.45 µm filtration: In a biosafety cabinet, a peristaltic pump, pump tubing, and a 0.45 µm membrane filter (PreFlow membrane filter capsules, Pall) were connected to the peristaltic pump. The 0.45 µm membrane filter was rinsed with sodium chloride injection at a flow rate of 20-200 mL/min, and finally the liquid was drained. P2 was filtered at a flow rate of 20-200 mL/min, and filtrate P3 was collected. The filter was removed, the tubing was washed with sodium chloride injection at a flow rate of 20-200 mL/min, and finally the liquid was drained and the pump tubing was detached.

S3 0.22 µm filtration: In a biosafety cabinet, a peristaltic pump, pump tubing, and a 0.22 µm membrane filter (Supor^{™} EX Grade ECV in Mini Kleenpak^{™} capsules, Pall) were connected to the peristaltic pump. The 0.22 µm membrane filter was rinsed with sodium chloride injection at a flow rate of 20-200 mL/min, and finally the liquid was drained. P3 was filtered at a flow rate of 20-200 mL/min, and the filtrate was collected to obtain plant exosomes. The filter was removed, the tubing was washed with sodium chloride injection at a flow rate of 20-200 mL/min, and finally the liquid was drained and the pump tubing was detached.

### 1.3 Isolation and extraction of plant exosomes using an ultracentrifugation method

The collected plant crude extract P1 was centrifuged using an ultracentrifuge at 120000 g, 4°C, for 70 min. After centrifugation was completed, the supernatant was removed, and the precipitate was resuspended with sodium chloride injection. After centrifugation was performed again at 120000 g, 4°C, for 70 min, the precipitate was resuspended with sodium chloride injection to obtain plant exosomes.

### 1.4 Transmission electron microscopy detection

Each of the plant exosome samples prepared above was taken in an amount of 5 uL and dropped onto a copper grid, followed by incubation at room temperature for 5 min; after incubation was completed, excess liquid was blotted from one side with absorbent paper; one drop of 2% uranyl acetate was added dropwise onto the copper grid, followed by incubation at room temperature for 1 min; after incubation was completed, excess liquid was blotted from one side with absorbent paper; and drying was carried out at room temperature for about 20 min. Transmission electron microscopy (Transmission Electron Microscopy, TEM) detection was performed on a nano transmission electron microscope (FEI, Tecnai G2 Spirit BioTwin).

### 1.5 Nanoparticle tracking analysis

Nanoparticle tracking analysis (NTA) technology was used to measure the particle size and concentration of each of the plant exosomes prepared above: a stock solution of a standard was diluted 1,000-fold to prepare a calibration stock solution (which may be prepared in proportion, with 1 µL of the standard stock solution used to prepare 1 mL of the calibration stock solution). 100 µL of the calibration stock solution prepared above was added to 25 mL of pure water to dilute it into a 250,000-fold calibration solution. When testing a sample, the sample was generally first diluted 1000-fold. After the instrument (Particle Metrix, PMX120) was calibrated with the calibration stock solution, the test sample was injected into the sample cell using a syringe, the sample concentration was measured, and three replicates were performed.

### 1.6 In vitro labeling of plant exosomes with PKH67

1 mM PKH67 (green fluorescent tracer dye) was diluted 50-fold to 20 µM with Diluent C (universal membrane labeling diluent). Subsequently, 5 µL of 20 µM PKH67 and 5 µL of the plant exosomes prepared above were each taken and mixed, followed by incubation at room temperature for 15 min. The positive rate of PKH67 in said plant exosomes was detected using a nano flow cytometer (Flow NanoAnalyzer).

### 1.7 Microprotein detection

40% SDS was prepared with pure water and filtered, and then diluted with pure water to 2% SDS as a standard curve diluent. According to the instructions of the Micro BCA^{™} protein assay kit (Thermo Fisher Scientific), reagents MA:MB:MC = 25:24:1 were mixed to prepare working reagent WR (Working Reagent, WR). A standard curve of 200, 40, 20, 10, 5, 2.5, 1, 0.5, and 0 µg/mL was prepared from a 2.0 mg/mL BSA stock solution using the diluent. After the plant exosomes were diluted with the diluent, an equal volume of WR was added, and constant-temperature shaking was performed at 100 rpm and 37°C for 2 hours. The absorbance of the standards and samples at 570 nm was detected with a microplate reader, and the protein concentration of the samples was calculated.

### 1.8 TNF-α inhibition rate detection

RAW264.7 cells (mouse mononuclear macrophage leukemia cells) were cultured in DMEM basal medium supplemented with 10% FBS for 48 h, and then cells were seeded in a 96-well plate at a density of 1.875×10⁴/cm². The cells were cultured in an incubator at 37.0°C and 5% CO₂ for 24 h. After plant exosomes (1×10⁹ particles/mL) and dexamethasone (final concentration 1 µg/mL) were used for pretreatment for 24 h, LPS (final concentration 5 ng/mL) was added for co-treatment for 4 h. The cell supernatant was collected and centrifuged at 500 g and 4°C for 5 min. The concentration of TNF-α was detected using a Mouse TNF-α ELISA kit (R&D systems), and the inhibition rate was calculated: 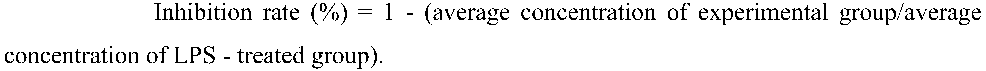 Inhibition rate (%) = 1 - (average concentration of experimental group/average concentration of LPS - treated group).

### 1.9 Sample preparation for non-targeted metabolomics analysis of plant exosomes

100 µL of a plant exosome liquid sample was pipetted into a 1.5 mL centrifuge tube, and 400 µL of extraction solution (acetonitrile:methanol = 1:1, containing 0.02 mg/mL internal standard L-2-chlorophenylalanine) was added; after vortex mixing for 30 s, low-temperature ultrasonic extraction was performed for 30 min (5°C, 40 KHz), and the sample was allowed to stand at -20°C for 30 min. Centrifugation was performed at 4°C and 13000 g for 15 min, the supernatant was transferred and dried under nitrogen, and the residue was reconstituted with 100 µL of reconstitution solution (acetonitrile:water = 1:1); low-temperature ultrasonic extraction was performed for 5 min (5°C, 40 KHz), centrifugation was performed at 4°C and 13000 g for 10 min, and the supernatant was transferred to an injection vial with an insert for instrumental analysis. Equal volumes of metabolites from all samples were mixed to prepare a quality control sample (Quality control, QC), and during instrumental analysis, one QC sample was inserted every 5-15 samples to examine the repeatability of the entire analysis process.

### 1.10 LC-MS/MS analysis

Equal volumes of metabolites from all samples were mixed to prepare a quality control sample, and during instrumental analysis, one QC sample was inserted every 5-15 samples to examine the repeatability of the entire analysis process. Mass spectrometry signal acquisition of the samples was performed in positive and negative ion scanning modes, with a mass scan range of 70-1050 m/z. The sheath gas flow rate was 50 psi, the auxiliary gas flow rate was 13 psi, the auxiliary gas heating temperature was 425°C, the ion spray voltage in positive mode was set to 3500 V, the ion spray voltage in negative mode was set to -3500 V, the ion transfer tube temperature was 325°C, and the normalized collision energy was 20-40-60 V stepped collision energy. The resolution of primary mass spectrometry was 60000, the resolution of secondary mass spectrometry was 7500, and data were acquired using DDA mode.

### 1.11 Substance identification and analysis

After instrumental analysis was completed, the LC-MS raw data were imported into metabolomics processing software Progenesis QI (Waters Corporation, Milford, USA) for baseline filtering, peak identification, integration, retention time correction, and peak alignment, thereby finally obtaining a data matrix of retention time, mass-to-charge ratio, and peak intensity; meanwhile, MS and MSMS mass spectrometry information was matched with public metabolite databases HMDB (http://www.hmdb.ca/) and Metlin (https://metlin.scripps.edu/), as well as the Majorbio self-built database, to obtain metabolite information.

The data matrix after database searching was uploaded to the Majorbio Cloud Platform (cloud.majorbio.com) for analysis. First, the data matrix was preprocessed as follows: missing values were removed from the data matrix using the 80% rule, that is, variables with non-zero values in more than 80% of at least one group of samples were retained, followed by filling missing values (missing values were filled with the minimum value in the original matrix); to reduce errors caused by sample preparation and instrument instability, the response intensities of sample mass spectrometry peaks were normalized using the total-sum normalization method, thereby obtaining a normalized data matrix. At the same time, variables with a relative standard deviation (RSD) > 30% in QC samples were deleted, and log10 logarithmic processing was performed to obtain a final data matrix for subsequent analysis.

Second, the ropls package (Version 1.6.2) in the R language was used to perform principal component analysis (PCA) and orthogonal partial least squares discriminant analysis (OPLS-DA) on the preprocessed data matrix, and 7-cycle cross-validation was used to evaluate model stability. Selection of significantly differential metabolites was determined based on variable importance in projection (VIP) values obtained from the OPLS-DA model and student's t-test p values, and metabolites with VIP > 1 and p < 0.05 were regarded as significantly differential metabolites.

Differential metabolites were subjected to metabolic pathway annotation through the KEGG database (Kyoto Encyclopedia of Genes and Genomes, https://www.kegg.jp/ kegg/pathway.html) to obtain pathways in which the differential metabolites participated. Pathway enrichment analysis was performed using the Python software package scipy.stats, and biological pathways most relevant to the experimental treatment were obtained through Fisher's exact test.

### 2. Experimental results and analysis

### 2.1 Screening of extraction methods

For Dendrobium, on the basis of Example 1.1 above, the following different filtration treatment combinations were performed, and the final results were evaluated. The specific extraction methods and corresponding results are shown in the following table:

| **Extraction method** | **2-30 µm deep filtration** | **0.45 µm filtration** | **0.22 µm filtration** | **Result evaluation** |
|---|---|---|---|---|
| **1** | - | - | + | Too many impurities, membrane clogging |
| **2** | - | + | - | Too many impurities, membrane clogging |
| **3** | + | - | - | Deep filtration has a strong dirt holding capacity and can completely filter the sample; the particle size is within the range, but sterility cannot be ensured. |
| **4** | + | - | + | Deep filtration has a strong dirt-holding capacity and can completely filter the sample, and the particle size is within the range; the 0.22 µm sterilizing filtration membrane is clogged. |
| **5** | + | + | - | Deep filtration has a strong dirt-holding capacity and can completely filter the sample, and the particle size is within the range; 0.45 µm filtration further removes some large-particle EVs, but sterility cannot be ensured. |
| **6** | + | + | + | Deep filtration has a strong dirt-holding capacity and can completely filter the sample, and the particle size is within the range; 0.45 µm filtration further removes some large-particle EVs, and 0.22 µm sterilizing filtration ensures sterility. The entire process is smooth and the yield is relatively high. |

| | | | | |
|---|---|---|---|---|
| Herein, "+" represents inclusion of the relevant treatment, and "-" represents absence of the relevant treatment. | | | | |

### 2.2 Extraction of plant exosomes (PEN) using layer-by-layer filtration and ultracentrifugation, respectively

Exosomes were isolated and extracted from Dendrobium officinale using the layer-by-layer filtration method in Examples 1.1-1.2. Specifically, Dendrobium officinale (up to 1000 g) was first subjected to mixing (treated by adding the solvent sodium chloride injection according to the mass-volume ratio (g/mL) shown in Table 1), pulverization, and stirring, so as to promote release of plant exosomes into the solvent sodium chloride injection. Plant residues were removed by centrifugation at 4000 g for 15 min to obtain plant crude extract P1. Larger impurities in P1 were removed by S1 (deep filtration) to obtain P2; larger particles in P2 were removed by prefiltration through S2 (0.45 µm membrane filtration) to obtain P3; and sterilizing filtration was performed through S3 (0.22 µm membrane filtration), thereby finally harvesting sterile-grade exosomes. On average, 500 mL of plant crude extract P1 took about 1 h.

Exosomes were isolated and extracted from Dendrobium officinale using the ultracentrifugation method in Example 1.3, mainly by centrifuging plant crude extract P1 twice at 12000 g for 70 min under conditions of 4°C. On average, 500 mL took about 3 h.

**Table 1 Name labels of PEN isolated from dendrobium officinale using ultracentrifugation and layer-by-layer filtration**

| **Plant category** | **Ratio** | **Layer-by-layer filtration** | **Ultracentrifugation** |
|---|---|---|---|
| Dendrobium officinale | 1:15 | SHV01 | DeE01 |

Each plant exosome sample obtained by the above operations was subjected to the following detections. The results show that the exosomes have the various characteristics and effects described above in the Detailed Description of the Invention section. As a representative example, various detection results of exosomes (name labels are shown in Table 1) obtained from 100 g of Dendrobium officinale by the above treatment are given below.

### 2.3 Physical characteristics of plant exosomes

After NTA detection was performed according to Example 1.5, the particle concentration, particle size, and final volume of the plant exosomes obtained in Example 2.2 above were statistically analyzed.

As shown in Table 2, the particle sizes of plant exosomes isolated and extracted by the layer-by-layer filtration method and the ultracentrifugation method were both within the range of 30-200 nm, but the yield of plant exosomes extracted by layer-by-layer filtration was significantly higher than the yield of exosomes extracted by ultracentrifugation. The results show that, in terms of both the efficiency of the exosome isolation method and the final harvested yield, the layer-by-layer filtration method is significantly superior to ultracentrifugation.

**Table 2 Particle size and concentration of plant exosomes**

| **Classification** | **Layer-by-layer filtration** | | | | **Ultracentrifugation** | | | |
|---|---|---|---|---|---|---|---|---|
| | **Particle concentration (×10¹⁰/mL)** | **Volume (mL)** | **Particle size (nm)** | **Yield based on 100 g (×10¹⁰)** | **Particle concentration (×10¹⁰/mL)** | **Volume (mL)** | **Particle size (nm)** | **Yield based on 100 g (×10¹⁰)** |
| **Dendrobium** | 0.7 | 1300 | 158.9 | 910 | 6.2 | 2 | 166.8 | 12.4 |

### 2.4 Qualitative metabolomics analysis of plant exosomes

### 2.4.1 Analysis of unique metabolites

Plant metabolomics analysis performed according to Examples 1.9 to 1.11 showed that, in terms of the number of metabolites identified in the Dendrobium officinale exosomes obtained in Example 2.2, layer-by-layer filtration was higher than ultracentrifugation. Specifically, as shown in Table 3. After Venn diagram analysis, the number of metabolites unique to layer-by-layer filtration was also higher than that of ultracentrifugation.

**Table 3 Statistical table of Venn analysis of exosomes extracted from dendrobium officinale using layer-by-layer filtration and ultracentrifugation**

| **Group** | **Layer-by-layer filtration Total metabolites** | **Ultracentrifu gation Total metabolites** | **Layer-by-layer filtration and Ultracentrifugation Metabolite intersection** | **Layer-by-layer filtration Unique metabolites** | **Ultracentrifu gation Unique metabolites** |
|---|---|---|---|---|---|
| Dendrobium | 1662 | 1493 | 1394 | 268 | 99 |

### 2.4.2 Compound classification analysis

Compound classification analysis is performed by classifying metabolites into sugars, amino acids, organic acids, lipids, and the like according to their structures and properties. According to the Superclass of the HMDB compound classification hierarchy, metabolites in Dendrobium officinale exosomes are mainly concentrated in six classes, namely lipids and lipid-like molecules, organic oxygen compounds, phenylpropanoids and polyketides, organoheterocyclic compounds, organic acids and derivatives thereof, and benzenoids. As shown in FIGs. 2-3.

The results show that, compared with DeE01, the additional metabolite classifications in SHV01 are phenylpropanoids and polyketides (57), lipids and lipid-like molecules (46), organic oxygen compounds (33), organoheterocyclic compounds (20), and organic acids and derivatives thereof (10).

### 2.4.3 Lipid classification analysis

Lipids and lipid-like molecules rank first in compound classification, and lipid classifications mainly include fatty acyls (FA), glycerolipids (GL), glycerophospholipids (GP), prenol lipids (PR), sphingolipids (SP), steroids (ST), and saccharolipids (SP).

Lipids and lipid-like molecules of Dendrobium officinale exosomes were classified and statistically analyzed. As shown in Table 4, the number of lipids in plant exosomes extracted by layer-by-layer filtration was greater than that extracted by ultracentrifugation. Compared with DeE01, SHV01 showed relatively large content differences in FA and PR, respectively.

**Table 4 Comparison of lipid classification of dendrobium officinale exosomes**

| **Plant classification** | **Exosome abbreviation** | | **FA** | **GL** | **GP** | **PR** | **SP** | **ST** | **SL** |
|---|---|---|---|---|---|---|---|---|---|
| Dendrobium | SHV01 | 237 | 4 | 4 | 284 | 3 | 27 | 2 | Dendrobium |
| | DeE01 | 229 | 6 | 7 | 243 | 3 | 24 | 2 | 0 |

### 2.5 Analysis of differential metabolites between plant exosomes

Differential metabolites were jointly screened using univariate statistical analysis (t-test) in combination with multivariate statistical analysis (OPLS-DA/PLS-DA) and fold change (FC) values, with screening conditions of P < 0.05, VIP > 1, and (FC < 1 or FC > 1, with FC not screened by default) [6].

As shown in FIG. 4, compared with DeE01, SHV01 had a total of 770 significantly differential metabolites, among which 537 metabolites were upregulated and 233 metabolites were downregulated.

### 2.6 Analysis of differential metabolic pathways between plant exosomes

Generally, a plurality of functionally interrelated metabolites constitute a metabolic pathway, and accumulation of expression differences of a plurality of metabolites in the metabolic pathway constitutes expression variation of the entire metabolic pathway. If the proportion of differential metabolites participating in a pathway is far greater than the proportion of background metabolites participating in this pathway, the experimental treatment is considered to be related to changes in this metabolic pathway. During enrichment analysis, based on the KEGG database, pathways with significant enrichment were screened according to an enrichment P value of less than 0.05.

Compared with DeE01, SHV01 had 73 pathways related to differential metabolites, among which a total of 20 pathways were significantly enriched.

As shown in FIG. 5, compared with ultracentrifugation, the differential metabolites of plant exosomes extracted by layer-by-layer filtration were mainly upregulated in Metabolism, and the main pathways included tryptophan metabolism, arachidonic acid metabolism, phenylpropanoid biosynthesis, starch and sucrose metabolism; alanine, aspartate and glutamate metabolism; galactose metabolism, ABC transporters, valine, leucine and isoleucine biosynthesis; cutin, suberine and wax biosynthesis; linoleic acid metabolism, β-alanine metabolism, nucleotide metabolism, histidine metabolism, aminoacyl-tRNA biosynthesis, arginine biosynthesis, and biosynthesis of various secondary metabolites.

### 2.7 TEM identification of plant exosomes isolated by layer-by-layer filtration

Analysis of the particle concentration, particle size, yield, and plant metabolomics of the above plant exosomes indicates that the layer-by-layer filtration extraction method is superior to ultracentrifugation and can extract a higher yield of plant exosomes in less time (equivalent to at least 40 times the yield of exosomes extracted by ultracentrifugation). Therefore, we further performed TEM on the plant exosomes extracted by layer-by-layer filtration.

As shown in FIG. 6, the Dendrobium officinale exosomes have a typical exosome-like saucer shape, with a particle size in the range of 30-200 nm (see FIG. 7), conforming to the definition of extracellular vesicles by the International Society for Extracellular Vesicles (ISEV).

### 2.8 PKH67 detection of plant exosomes isolated by layer-by-layer filtration

The plant exosomes prepared in Example 2.2 above were stained in vitro using the lipophilic dye PKH67, and PKH67-positive particles were detected and analyzed by a nano flow cytometer.

### As shown in FIG. 8, the positive rate of PKH67 of SHV01 was 97.4%.

### 2.9 Detection of protein content of plant exosomes isolated by layer-by-layer filtration

The protein content of plant exosomes was detected, and it was found that the protein content of Dendrobium officinale exosomes was abundant. Specifically, as shown in FIG. 9A, SHV01 was 875.89 µg/mL.

### 2.10 Detection of anti-inflammatory activity of plant exosomes isolated by layer-by-layer filtration

The efficacy of the plant exosomes extracted by layer-by-layer filtration was detected, mainly by observing the inhibition rate of inhibiting LPS-induced TNF-α release from RAW264.7 for evaluation.

As shown in FIG. 9B, the TNF-α inhibition rate of the positive control group, 1 µg/mL dexamethasone, was 91.41%. When Dendrobium officinale exosomes with a particle concentration of 1×10⁹/mL were added, the TNF-α inhibition rate of SHV01 was 64.40%.

The results indicate that the Dendrobium officinale exosomes extracted by layer-by-layer filtration have satisfactory anti-inflammatory and immunomodulatory activities.

### 2.11 Scale-up process

According to the layer-by-layer filtration method in Examples 1.1-1.2, 500 g of Dendrobium officinale was treated to isolate and extract exosomes, so as to verify whether layer-by-layer filtration could process plant raw material having a mass of about 1000 g or even more. After NTA detection was performed according to Example 1.5, the particle concentration, particle size, and final volume of the obtained plant exosomes were statistically analyzed.

As shown in Table 5, after scale-up by 5-10 times compared with the 100 g plant raw material from which exosomes were obtained in Example 2.3 (for example, Dendrobium was scaled up by 5 times), about 5000 mL of plant crude extract could be processed by increasing the membrane area in the layer-by-layer filtration method. The particle sizes of the isolated and extracted plant exosomes were all within the range of 30-200 nm, and the final harvested exosome yield was also relatively considerable.

**Table 5 Particle size and concentration of plant exosomes after 5-10 times scale-up of layer-by-layer filtration process**

| **Classification** | **Plant raw material (g)** | **Volume (mL)** | **Particle concentration (×10¹⁰/mL)** | **Particle size (nm)** | **Total exosome yield (×10¹⁰)** |
|---|---|---|---|---|---|
| **Dendrobium** | 500 | 4770 | 0.59 | 112.8 | 2814.3 |

### 2.12 Isolation and identification of plant extracellular vesicles

### Isolation of plant extracellular vesicles

We used the layer-by-layer filtration method of Examples 1.1-1.2 to isolate extracellular vesicles from Dendrobium officinale, Rehmannia glutinosa, and Angelica sinensis, respectively (named SH, DH, and DG, respectively).

### Identification of plant extracellular vesicles

The extracellular vesicles isolated from the three plants all had a typical cup-shaped morphology (FIGs. 10A, D, and G). NTA showed that the size of SH was 163.1±7.8 nm (FIG. 10B), the size of DH was 127.9±5.9 nm (FIG. 10E), and the size of DG was 125.1±7.2 nm (FIG. 10H). After the three plant extracellular vesicles were labeled with PKH67, they were detected using NTA. The PKH67-positive rate of SH was 97.4% (FIG. 10C), the PKH67-positive rate of DH was 34.7% (FIG. 10F), and the PKH67-positive rate of DG was 44.0% (FIG. 10I). The above results indicate that an extracellular vesicle-like plant small vesicle can be extracted from Dendrobium officinale, Rehmannia glutinosa, and Angelica sinensis using the layer-by-layer filtration method.

### 2.13 Anti-aging effect of plant extracellular vesicles on fibroblasts

Ultraviolet irradiation can affect gene expression in fibroblasts and reduce collagen synthesis, thereby disrupting the balance between collagen synthesis and degradation in the extracellular matrix (ECM). Among the many types of collagen, type I collagen accounts for 80% of adult skin and is mainly responsible for the supporting function of skin, but the synthetic ability of type I collagen itself gradually weakens with aging or external factors such as ultraviolet rays. Therefore, we detected whether SH, DH, and DG could promote synthesis of type I collagen in mouse embryonic fibroblasts (3T3 cells, from the Cell Bank of the Chinese Academy of Sciences, catalog No. SCSP-515) by Western Blot technology.

The experimental results showed that, compared with the control group, after 3T3 cells were cultured with SH at a concentration of 1×10⁸ particles/mL for 24 h, expression of type I collagen was significantly upregulated, and the overall effect was superior to 5 ng/mL TGF-β (positive drug). DH and DG had certain effects of promoting type I collagen expression, but the effects were less significant (FIG. 11).

After multiple repeated experiments and grayscale analysis of protein bands using ImageJ, we calculated the increase rate of type I collagen expression and found that, at a concentration of 1×10⁸ particles/mL, the average promotion rate of SH on basal type I collagen expression in 3T3 cells could reach more than 50%, which was obviously superior to the positive drug TGF-β at 5 ng/mL (about 27%), DH (29.33%), and DG (19.27%) (Table 6). In summary, the experimental results indicate that different plant extracellular vesicles subjected to deep filtration have different anti-aging effects, among which Dendrobium officinale has the most obvious anti-aging effect and is superior to the positive control group.

**Table 6 Promotion Rate of Plant Extracellular Vesicles on Basal Collagen Level in 3T3 Cells**

| **Replicate experiment** | **TGF-β** | **SH** | **DH** | **DG** |
|---|---|---|---|---|
| **First** | 71.81% | 95.63% | 56.67% | 53.29% |
| **Second** | 5.11% | 29.33% | 13.29% | 2.77% |
| **Third** | 5.13% | 31.92% | 18.03% | 1.77% |
| **Average** | **27.35%** | 52.30% | 29.33% | 19.27% |

### 2.14 Anti-aging effects of active ingredients of dendrobium officinale obtained by different extraction processes on fibroblasts

### Preparation of active ingredients of dendrobium officinale

We further compared the anti-aging effects on fibroblasts of Dendrobium officinale extracellular vesicles (SH) obtained by layer-by-layer filtration (the extraction method is as described in Example 1.12), Dendrobium officinale extracellular vesicles (UC) obtained by ultracentrifugation, and Dendrobium officinale polysaccharides obtained by alcohol extraction (EE, 80% Dendrobium officinale polysaccharides, purchased from Xi'an Ruihe Biotechnology Co., Ltd.). UC was obtained by washing Dendrobium officinale, slicing and crushing it in a juicer, and collecting the juice; filtering the juice through a 100-mesh screen to remove impurities and collecting the filtrate; centrifuging the filtrate at 2000-4500 g for 5-20 min, discarding the precipitate, and collecting the supernatant; then centrifuging the supernatant at 8000-11000 g for 25-40 min, discarding the precipitate, and collecting the supernatant; and finally centrifuging the supernatant at 100000-150000 g and 4°C for 70-100 min, and collecting the precipitate to obtain Dendrobium officinale extracellular vesicles.

### Detection of anti-aging effect on fibroblasts

Ultraviolet stimulation can induce cellular senescence, and one of the significant characteristics of senescent cells is a change in cell morphology; senescent cells usually become enlarged, which is often regarded as a hallmark molecular event of UV-induced cellular senescence. Phalloidin is a toxic cyclic heptapeptide obtained from poisonous Amanita phalloides fungi, can selectively bind to actin in animals and plants, and is a staining tool for the cytoskeleton. Under an optical microscope, fluorescently labeled phalloidin can clearly show the morphology and distribution of intracellular microfilaments. Therefore, we detected the effect of UVA irradiation on the morphology of human embryonic lung fibroblasts (HSF cells, purchased from the Kunming Cell Bank of the Chinese Academy of Sciences, catalog No. KCB 200537) by fluorescently labeled phalloidin.

### Experimental results

Through modeling, we found that after HSF cells were stimulated with UVA at a dose of 5 J/cm² for 24 h, the cells exhibited a flattened and enlarged senescence-like morphology (FIGs. 12A-B), which was consistent with literature reports. Based on this morphological criterion, we counted enlarged cells after UVA irradiation under a fluorescence microscope.

The experimental results showed that after HSF cells were stimulated with UVA at a dose of 5 J/cm² for 24 h, the proportion of enlarged cells increased from 3.78% to 12.57%; compared with the 5 J/cm² UVA model group, after treatment with 1×10⁸ particles/mL SH, UC, and 0.025% (v/v) EE added before and after UVA irradiation, respectively, the proportions of enlarged cells decreased from 12.57% to 4.82%, 8.13%, and 9.89%, respectively (FIGs. 12C-D, Table 7).

These results indicate that both SH and UC can significantly improve UVA-induced cell enlargement, among which SH has the best effect and can basically restore cell morphology to the normal level.

**Table 7 Proportion of enlarged cells in each experimental group**

| **F-actin staining** | **Con** | **UVA** | **SH** | **UC** | **EE** |
|---|---|---|---|---|---|
| **First** | 3.69% | 10.30% | 4.63% | 8.42% | 8.26% |
| **Second** | 3.64% | 13.10% | 4.89% | 7.96% | 11.00% |
| **Third** | 4.02% | 14.30% | 4.95% | 8.00% | 10.40% |
| **Average** | 3.78% | **12.57%** | **4.82%** | **8.13%** | **9.89%** |

In addition, Western Blot results showed that, compared with the control group, after the SH and UC treatment groups were cultured with 3T3 cells for 24 h, type I collagen expression was significantly upregulated, among which the effect of SH was superior to 5 ng/mL TGF-β (positive drug). No effect of EE in promoting type I collagen expression was observed (FIG. 13). After multiple repeated experiments and grayscale analysis of protein bands using ImageJ, we calculated the increase rate of type I collagen expression and found that the promotion rate of the positive drug TGF-β at 5 ng/mL on basal type I collagen expression in 3T3 cells was about 53.7%, whereas the average promotion rate of SH at a concentration of 1×10⁸ particles/mL on basal type I collagen expression in 3T3 cells could reach nearly 80%, which was obviously superior to the positive drug as well as UC (54.27%) and the EE group (9.22%) (Table 8). In summary, the experimental results indicate that, compared with different extraction processes, Dendrobium officinale extracellular vesicles subjected to layer-by-layer filtration have the best anti-aging effect.

**Table 8 Promotion rate of each experimental group on type I collagen synthesis in 3T3 fibroblasts**

| **WB experiment** | **TGF-β** | **SH** | **UC** | **EE** |
|---|---|---|---|---|
| **First** | 89.84% | 118.19% | 65.69% | -2.05% |
| **Second** | 52.98% | 64.17% | 56.06% | 6.34% |
| **Third** | 18.28% | 54.85% | 41.05% | 23.37% |
| **Average** | **53.70%** | **79.07%** | **54.27%** | **9.22%** |

### 2.15 Metabolomics analysis of dendrobium officinale extracellular vesicles obtained by different isolation methods

In order to compare differences in metabolites contained in Dendrobium officinale extracellular vesicles isolated by the layer-by-layer filtration method and the ultracentrifugation method in Example 2.14, we used untargeted metabolomics for analysis. After data preprocessing, 1662 metabolites were identified in SH, and 1493 metabolites were identified in UC. Venn diagram analysis showed that SH and UC had 1394 common metabolites, SH had 268 unique metabolites, and UC had 99 unique metabolites (FIG. 14).

The expression levels of total metabolites in SH were subjected to Log transformation, the top 100 metabolites were screened according to the average content of the two replicate groups of metabolites, and cosmetic efficacy analysis of the metabolites was performed according to compound classification (https://pubmed.ncbi.nlm.nih.gov/); the results are shown in Table 9. Among them, there were 17 skin-related metabolites, which were classified as hydroxy acids and derivatives thereof, keto acids and derivatives thereof, fatty acyls, organic oxygen compounds, flavonoids, carboxylic acids and derivatives thereof, phenols, prenol lipids, benzenes and substituted derivatives. The possible effects exerted in cosmetics mainly include moisturizing, antioxidation, promoting collagen synthesis, repairing the skin barrier, anti-photoaging, anti-aging, and the like. Dendrobium officinale contains trace elements and minerals required by skin during metabolism; after these substances are absorbed, they can promote skin metabolism and can also promote discharge of some metabolites such as oxygen free radicals, further supporting that SH has effects of anti-photoaging, restoring skin elasticity, and removing wrinkles.

**Table 9 Representative metabolites of SH (sorted by content)**

| **Classification** | **Representative substance** |
|---|---|
| Hydroxy acids and derivatives thereof | Malic acid (3) |
| Keto acids and derivatives thereof | α-ketoglutaric acid (9) |
| Fatty acyls | Palmitic acid (5) |
| | 10-hydroxystearic acid (11) |
| Organic oxygen compounds | Trehalose (2) |
| | Fructan (14) |
| | Sucrose (44) |
| | Raffinose (46) |
| Flavonoids | Rutin (6) |
| | Quercetin (53) |
| | Isoquercetin (70) |
| Carboxylic acids and derivatives thereof | Citric acid (68) |
| | Proline (79) |
| | Pantothenic acid (88) |
| Phenols | Zingerone (63) |
| Prenol lipids | Ursolic acid (66) |
| Benzene and substituted derivatives | p-aminobenzoic acid (72) |

### 2.16 Transdermal Permeability of SH

To verify whether SH can penetrate through the stratum corneum and epidermal layer to exert its active effect, we performed a transdermal experiment using a Franz diffusion cell based on suckling pig skin, detected the protein content in the receiving chamber at different time points, and calculated the cumulative permeation amount and diffusion percentage (FIG. 15).

According to the experimental results, the permeation rate of SH within 24 hr was 0.0039 mg/h (FIG. 15A), and the diffusion percentage reached 82.55% (FIG. 15B), indicating that permeation occurred.

### 2.17 Firming and anti-wrinkle effects of SH on a 3D full-thickness skin model

We used a 3D full-thickness skin model (FuKutis^{®}, Guangdong Boxi Biotechnology Co., Ltd.) to simulate aging damage of skin caused by UV irradiation through UVA (irradiation dose 35 J/cm²), and used 1.1×10⁹ particles/mL SH concentration, a 0.9 mL loading volume, and a total administration amount of 9.9×10⁹ particles to perform submerged administration on the skin model; changes in tissue structure and collagen fibers under different treatment conditions were observed by section staining.

As shown in FIG. 16, by H&E and Masson staining, we observed that the epidermal layer of the UV-irradiated model was significantly thinned (FIG. 16A), and the collagen fiber content was significantly decreased (FIG. 16B). Statistics of the thickness of the viable epidermal cell layer showed that, after SH treatment, compared with the UV group, the thickness of the viable epidermal cell layer was significantly increased, with an increase rate of 37.63% (FIG. 16C); the collagen fiber content in the SH treatment group was increased by 241.07% compared with the UV group (FIG. 16D).

Skin elasticity is the ability of skin to stretch and return to its original shape. A skin elasticity tester (Cutometer^{®} Dual MPA580) determines the elastic properties of skin based on the principles of suction and stretching; the higher the elasticity coefficient R2 (%) value, the better the skin elasticity, whereas a lower value indicates looser skin. Based on the experimental results of tissue structure and collagen fibers, we also detected the skin elasticity of the 3D skin.

The experimental results showed that the R2 value of the skin model treated with SH was significantly increased by 41.49% (FIG. 16E).

In summary, SH can significantly improve thinning of the viable epidermal cell layer and reduction of collagen fibers in the 3D full-thickness skin model caused by UV irradiation (FIGs. 16A-D), and also plays a significant role in maintaining skin elasticity (FIG. 16E).

### 2.18 Regulatory effect of SH on collagen expression

To further study the regulatory effect of SH on collagen expression, we sectioned the 3D full-thickness skin model in Example 2.17, performed specific immunofluorescence detection for type I collagen (COL I, FIG. 17A) and type IV collagen (COL IV, FIG. 17B), and statistically analyzed protein contents (FIGs. 17C-D) by integrated optical density (Integrated Optical Density, IOD). COL I accounts for about 80% of collagen in the dermis of skin and forms collagen fibers with COL III; promoting COL I can achieve a certain effect of resisting wrinkles and aging, making the skin plump and full. COL IV is mainly a key protein at the dermal-epidermal junction and is an important element supporting the skin.

The results showed that the UV-irradiated model clearly exhibited reductions of COL I in the dermis (FIG. 17A) and COL IV at the dermal-epidermal junction (FIG. 17B), whereas SH treatment significantly enhanced expression of both COL I (FIG. 17C) and COL IV (FIG. 17D), with increase rates of 133.90% and 118.00%, respectively.

### 2.19 Effects of SH on UV-induced cellular senescence and hyaluronic acid (HA) expression

DNA damage can be caused by internal and external factors affecting the body, such as exposure to exogenous UV irradiation, which causes DNA damage and thereby induces aging of the body. When DNA is irradiated by UV, cyclobutane-pyrimidone dimers (CPD) and pyrimidine 6-4 pyrimidone photoproducts (6-4PP) are mainly produced. By detecting CPD through specific immunohistochemistry, we found that CPD expression was significantly increased in the UV-irradiated model, and SH could significantly reduce the rate of CPD-positive cells (FIGs. 18A and C).

HA has the highest content in skin and provides stability and elasticity of the extracellular matrix by moisturizing, maintaining extracellular spaces, and interacting with other substances, thereby resisting wrinkles, enhancing skin elasticity, and making the skin plump and full. UV radiation can significantly reduce the HA content in skin, whereas SH can significantly increase the HA content (FIGs. 18B and D).

### 2.20 Mechanistic study of firming and anti-wrinkle effects of SH

We further investigated the firming and anti-wrinkle mechanism of SH. With respect to basement membrane-associated proteins, laminin (Laminin, LN) is an important component protein of the extracellular matrix (ECM) widely distributed in vivo and is an important component constituting the basement membrane. As a multifunctional adhesion molecule, LN participates in the synthesis process of ECM and is an important substance for regulating cell proliferation, growth, differentiation, apoptosis, migration, and infiltration. Nidogen exists in all basement membranes and is an important structural protein of the basement membrane. It binds to LN at a ratio of 1:1 to form a stable complex and promotes non-covalent molecular linkage between LN protein and COL IV. By detecting LN 5 (FIGs. 19A and C) and nidogen (FIGs. 19B and D) in the 3D skin model after UV radiation, we found that SH could significantly upregulate the contents of LN 5 and nidogen, thereby stabilizing the basement membrane structure and achieving firming and anti-wrinkle effects.

On the other hand, with respect to junction proteins, integrin (Integrin) α6β4 forms the central core of hemidesmosomes and connects the ECM with the keratin cytoskeletal network in keratinocytes, and is an important junction protein at the dermal-epidermal junction. Plectin also plays a crucial role in maintaining the integrity of cell structure. It is a component of desmosomal complexes connecting cytoskeletal proteins and transmembrane molecules. In epithelial cells, plectin connects cytokeratin and integrin α6β4 in hemidesmosomes to the extracellular matrix. By detecting integrin α6β4 (FIGs. 20A and C) and plectin (FIGs. 20B and D)in the 3D skin model after UV radiation, we found that SH could significantly upregulate the contents of integrin α6β4 and plectin, thereby strengthening the connection between the dermis and epidermis as well as the tightness of binding between cells and the ECM, so as to achieve firming and anti-wrinkle effects.

### REFERENCES

[1] Zhang M, Viennois E, Prasad M, Zhang Y, Wang L, Zhang Z, Han MK, Xiao B, Xu C, Srinivasan S, Merlin D. Edible ginger-derived nanoparticles: A novel therapeutic approach for the prevention and treatment of inflammatory bowel disease and colitis-associated cancer. Biomaterials. 2016 Sep; 101:321-40.
[2] Mu J, Zhuang X, Wang Q, Jiang H, Deng ZB, Wang B, Zhang L, Kakar S, Jun Y, Miller D, Zhang HG. Interspecies communication between plant and mouse gut host cells through edible plant derived exosome-like nanoparticles. Mol Nutr Food Res. 2014 Jul;58(7):1561-73.
[3] Baldini N, Torreggiani E, Roncuzzi L, Perut F, Zini N, Avnet S. Exosome-like Nanovesicles Isolated from Citrus limon L. Exert Antioxidative Effect. Curr Pharm Biotechnol. 2018;19(11):877-885.
[4] Mu N, Li J, Zeng L, et al. Plant-Derived Exosome-Like Nanovesicles: Current Progress and Prospects. Int J Nanomedicine. 2023; 18:4987-5009. Published 2023 Sep 5.
[5] Théry C, Witwer KW, Aikawa E, et al. Minimal information for studies of extracellular vesicles 2018 (MISEV2018): a position statement of the International Society for Extracellular Vesicles and update of the MISEV2014 guidelines. J Extracell Vesicles. 2018;7(1):1535750.
[6] Wang X, Li J, Zhang A H. Urine metabolic phenotypes analysis of extrahepatic cholangiocarcinoma disease using ultra-high performance liquid chromatography-mass spectrometry[J]. RSC Advances, 2016, 6(67): 63049-63057.
[7] CN108384743A.

## Claims

1. A method for isolating and extracting plant exosomes, specifically a method for isolating and extracting plant exosomes by deep filtration, wherein the method comprises the following steps:
(a) obtaining a plant material;
(b) pretreating the obtained plant material to obtain a plant crude extract solution; and
(c) subjecting the obtained plant crude extract solution to layer-by-layer filtration treatment,
wherein in step (c), the layer-by-layer filtration treatment comprises a deep filtration step, and optionally a membrane filtration step.

2. The method according to claim 1, wherein in step (c), at least one membrane filtration step is performed after the deep filtration step; preferably, the membrane filtration comprises anti-clogging membrane filtration, or further comprises sterilizing membrane filtration; more preferably, the membrane filtration consists of anti-clogging membrane filtration and sterilizing membrane filtration performed sequentially.

3. The method according to claim 2, wherein the anti-clogging membrane filtration has a pore size of about 0.4-0.8 µm, preferably about 0.45-0.8 µm, more preferably about 0.45 µm; and the sterilizing membrane filtration has a pore size of about 0.1-0.3 µm, preferably about 0.1-0.22 µm, more preferably about 0.22 µm.

4. The method according to any one of claims 1-3, wherein in step (c), the deep filtration employs deep filtration with a pore size of 1-50 µm, preferably deep filtration with a pore size of 2-30 µm, more preferably deep filtration with a pore size of 6-30 µm.

5. The method according to any one of claims 1-4, wherein in step (c), a loading capacity of the deep filtration is about 50-200 L/m², and a filtration flow rate is about 100-1000 LMH; and/or the deep filtration step is performed using a depth capsule filter, preferably a depth capsule filter having a dual-layer membrane structure.

6. The method according to any one of claims 1-5, wherein the plant material is selected from Dendrobium, preferably Dendrobium officinale.

7. The method according to any one of claims 1-6, wherein the method can process a plant raw material having a weight of at least about 100 g, 200 g, 300 g, 400 g, 500 g, 600 g, 700 g, 800 g, 900 g, 1000 g, 2000 g, 3000 g, 4000 g, 5000 g, 6000 g, 7000 g, 8000 g, 9000 g, 10000 g, 50000 g, or 100000 g; and/or the method increases the yield by at least about 40 times compared with an ultracentrifugation method.

8. A plant exosome, which is prepared by the method according to any one of claims 1-7 or is derived from Dendrobium, preferably Dendrobium officinale.

9. The plant exosome according to claim 8, having more metabolites in one or more of the following aspects compared with a plant exosome prepared by an ultracentrifugation method: lipids and lipid-like molecules, organic oxygen compounds, phenylpropanoids and polyketides, organoheterocyclic compounds, organic acids and derivatives thereof, and/or benzenoids.

10. A plant exosome composition, comprising the plant exosome according to claim 8 or 9, and optionally a suitable excipient or carrier.

11. The plant exosome composition according to claim 10, wherein the plant exosome composition is a pharmaceutical composition, a food composition, a beauty or cosmetic composition, or a nutrition and health-care composition.

12. Use of the plant exosome according to claim 8 or 9 or the plant exosome composition according to claim 10 or 11 in the preparation of a drug, food, nutrition and health-care product, or beauty or cosmetic product, wherein the product is used for treating or preventing inflammation, preferably skin inflammation, or helping or assisting in improving, alleviating, or controlling inflammation, preferably skin inflammation, or is used for or assists in providing repairing, anti-inflammatory, antioxidant, anti-aging, whitening, firming, anti-wrinkle, or moisturizing effects in the dermatological field.

13. The use according to claim 12, wherein the plant exosome is used for anti-aging effect on skin, such as anti-aging effect on fibroblasts, for example for improving or reducing skin aging induced by ultraviolet irradiation or UV-induced cellular senescence, or for, in skin cells (preferably UV-irradiated skin cells), promoting expression of type I collagen and/or type IV collagen, reducing cyclobutane-pyrimidone dimers (CPD), or increasing hyaluronic acid (HA).

14. The use according to claim 12, wherein the plant exosome is used for a firming and anti-wrinkle effect on skin, preferably the skin is subjected to UV irradiation, for example, the plant exosome is used for increasing or restoring viable epidermal layer thickness or increasing collagen fibers, improving thinning of the viable epidermal cell layer and reduction of collagen fibers in skin caused by UV irradiation, and/or maintaining skin elasticity, and further for upregulating the content of laminin (such as LN 5), nidogen, integrin α6β4 and/or plectin.

15. Use of the plant exosome according to claim 8 or 9 or the plant exosome composition according to claim 10 or 11 for treating or preventing inflammation, preferably skin inflammation, or helping or assisting in improving, alleviating, or controlling inflammation, preferably skin inflammation, or is used for or assists in providing repairing, anti-inflammatory, antioxidant, anti-aging, whitening, firming, anti-wrinkle, or moisturizing effects in the dermatological field.

16. The use according to claim 15, wherein the plant exosome is used for anti-aging effect on skin, such as anti-aging effect on fibroblasts, for example for improving or reducing skin aging induced by ultraviolet irradiation or UV-induced cellular senescence, or for, in skin cells (preferably UV-irradiated skin cells), promoting expression of type I collagen and/or type IV collagen, reducing cyclobutane-pyrimidone dimers (CPD), or increasing hyaluronic acid (HA).

17. The use according to claim 15, wherein the plant exosome is used for a firming and anti-wrinkle effect on skin, preferably the skin is subjected to UV irradiation, for example, the plant exosome is used for increasing or restoring viable epidermal layer thickness or increasing collagen fibers, improving thinning of the viable epidermal cell layer and reduction of collagen fibers in skin caused by UV irradiation, and/or maintaining skin elasticity, and further for upregulating the content of laminin (such as LN 5), nidogen, integrin α6β4 and/or plectin.

18. A method for treating or preventing inflammation, preferably skin inflammation, comprising administering the plant exosome composition according to claim 10 or 11 to a subject in need thereof.

19. A method used in dermatology for repairing, anti-inflammation, antioxidation, anti-aging, whitening, firming and anti-wrinkle, or moisturizing, comprising administering the beauty or cosmetic composition and/or nutrition and health-care composition according to claim 11 to a subject in need thereof.

20. A method for improving, alleviating, or controlling dermatological symptoms, comprising administering the plant exosome composition according to claim 10 or 11 to a subject in need thereof.
